(19) Europäisches Patentamt
European Patent Office
Office européen des brevets

(11) EP 4 498 389 A1

(12) EUROPEAN PATENT APPLICATION

(43) Date of publication:
**29.01.2025 Bulletin 2025/05**

(21) Application number: **24184149.3**

(22) Date of filing: **25.06.2024**

(51) International Patent Classification (IPC):
***G16H 50/20*** *(2018.01)* ***G16H 15/00*** *(2018.01)*
***A61B 5/00*** *(2006.01)* ***G16H 20/40*** *(2018.01)*
***G16H 40/40*** *(2018.01)* ***G16H 40/67*** *(2018.01)*
***G16H 50/30*** *(2018.01)* ***G16H 50/70*** *(2018.01)*

(52) Cooperative Patent Classification (CPC):
**G16H 15/00; A61B 5/00; G16H 20/40; G16H 40/40; G16H 40/67; G16H 50/20; G16H 50/30; G16H 50/70**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **25.07.2023 US 202363515359 P**
**20.06.2024 US 202418748167**

(71) Applicant: **Pacesetter, Inc.**
**Sylmar, CA 91342 (US)**

(72) Inventors:
- **LASHGARI, Elnaz**
**Anaheim Hills, CA (US)**
- **ADITYA, Goil**
**Stevenson Ranch, CA (US)**

(74) Representative: **Barker Brettell Sweden AB**
**Kungsbroplan 3**
**112 27 Stockholm (SE)**

(54) **CONVOLUTIONAL NEURAL NETWORK FOR AUTOMATIC DISCRIMINATION OF PAUSE EPISODES DETECTED BY AN IMPLANTABLE MEDICAL DEVICE**

(57) System (200, 700) and method for declaring pause in cardiac activity comprises memory (704) to store specific executable instructions and a convolutional neural network (CNN) model comprising a global average pooling (GAP) layer. One or more processors (702) are configured to execute the specific executable instructions to obtain device classified arrhythmia (DCA) data sets generated by an implantable medical device (IMD) (212, 703) for corresponding candidate pause episodes declared by the IMD (212, 703). The DCA data sets include cardiac activity (CA) signals for one or more beats sensed by the IMD (212, 703). The processor(s) (702) apply the CNN model to the DCA data sets to identify a valid subset of the DCA data sets that correctly characterizes the corresponding CA signals. A display is configured to present information concerning the valid subset of the DCA data sets.

Obtain Collection of Reference DCA Data Sets
502
Augment Collection of Reference DCA Data Sets
504
Sufficient Amount of Reference and Augmented DCA Data Sets?
506
No
Yes
Input Reference and Augmented DCA Data Sets into the CNN Model
508
Train CNN Model
510
Save CNN Model
512
514
Generate Personalized CNN Model
Train New CNN Model Using Transfer Learning
516

FIG. 5



Processed by Luminess, 75001 PARIS (FR)

## Description

FIELD OF THE INVENTION

Embodiments herein relate generally to confirming and/or treating episodes of pause in cardiac activity signals utilizing a convolutional neural network.

BACKGROUND OF THE INVENTION

Implantable medical devices (IMDs) can be implanted within a patient to monitor cardiac rhythms and in some cases, deliver therapy to treat a patient, such as with pacing pulses. Implantable cardiac monitors (ICMs), a type of IMD, are small, single-lead devices implanted subcutaneously within a patient to monitor cardiac rhythms for extended periods. ICMs automatically record arrhythmia events such as atrial fibrillation (AF) and pauses, as well as capture patient-activated symptom episodes. The recorded data is transmitted to a remote monitoring platform, which notifies clinicians for further analysis and intervention.

Today, numerous arrhythmia detection processes are implemented within IMDs, such as but not limited to ICMs, that detect arrhythmias based on various criteria, such as irregularities and variation patterns in R-wave to R-wave (RR) intervals. In some embodiments, the arrhythmia detection process steps beat by beat through cardiac activity (CA) signals and analyzes the characteristics of interest, such as RR intervals over a period of time. An arrhythmia episode is declared based on the characteristics of interest, such as when the RR interval pattern for the suspect beat segments is sufficiently irregular and dissimilar from RR interval patterns for sinus beat segments. When the ICM detects an arrhythmia episode, the ICM stores the CA signals (e.g., electrocardiograms or EGM signals) associated with the episode as an arrhythmia episode (AE) data set, and includes with the AE data set one or more device documented (DD) markers designating aspects of interest within the CA signals and/or episode.

However, arrhythmia detection processes at times may declare false arrhythmia episodes when a patient is not experiencing an arrhythmia. When a false arrhythmia episode is declared, the ICM continues to store the CA signals associated with the episode as an AE data set, with the DD markers (albeit incorrect/false DD marker). For example, false pause detection may arise due to various conditions, such as undersensing of R waves or transient loss of electrode tissue contact.

For certain implantable devices and conditions, large numbers of AE data sets may be stored and transmitted due to frequent false detections. This is particularly a challenge with ICMs, in which computational and power constraints of the ICMs limit the internal algorithms, which can lead to misclassification errors. False arrhythmia detections can result in additional review burden and resource utilization for clinics, who often must spend considerable time reviewing the AE data sets.

Also, for implantable devices that provide treatment, declaring a false arrhythmia episode when the patient is not experiencing an arrhythmia may lead to over treatment, which may negatively impact the patient. In some cases, an increase in treatment may use more battery power than needed, reducing the useable life of the IMD.

A need remains to reduce the burden placed on clinicians for reviewing EGM signals and DD markers, and in particular, EGM signals in connection with false arrhythmia episodes such as pause events, as well as to improve the treatment of patients.

SUMMARY

In accordance with embodiments herein, a system for declaring pause in cardiac activity comprises memory to store specific executable instructions and a convolutional neural network (CNN) model trained to detect pause episodes. The CNN model comprises a global average pooling (GAP) layer. The system includes one or more processors configured to execute the specific executable instructions to obtain device classified arrhythmia (DCA) data sets generated by an implantable medical device (IMD) for corresponding candidate pause episodes declared by the IMD, the DCA data sets including cardiac activity (CA) signals for one or more beats sensed by the IMD, and apply the CNN model to the DCA data sets to identify a valid subset of the DCA data sets that correctly characterizes the corresponding CA signals. The system includes a display configured to present information concerning the valid subset of the DCA data sets.

Optionally, the one or more processors are further configured to apply the CNN model to the DCA data sets to identify an invalid subset of the DCA data sets that incorrectly characterizes the corresponding CA signals, wherein the valid subset of the DCA data sets indicates a portion of the DCA data sets that indicates true pause, and the invalid subset of the DCA data sets indicates a second portion of the DCA data sets that indicates false pause.

Optionally, the information is indicative of a recommendation for i) a change in a treatment for a patient associated with the IMD that will increase the DCA data sets identified as the valid subset of the DCA data sets, or ii) a change in a treatment associated with the IMD that will increase the DCA data sets identified as the valid subset of the DCA data sets.

Optionally, the one or more processors are further configured to apply the CNN model to the DCA data sets to identify an invalid subset of the DCA data sets that incorrectly characterizes the corresponding CA signals, wherein in response to the change in the treatment for the patient or the IMD, the one or more processors are further configured to obtain second DCA data sets generated by the IMD, apply the CNN model to the second DCA data sets to identify a second invalid subset of the DCA data sets that incorrectly characterizes the corresponding CA signals, and confirm that the second invalid subset of the DCA data sets has fewer invalid candidate pause episodes compared to the invalid subset of the DCA data sets.

Optionally, the CNN model comprises at least two 1-dimensional (1D) convolutional layers and the GAP layer is configured to receive outputs from each of the at least two 1D convolutional layers, wherein the one or more processors are further configured to process an output of a first 1D convolutional layer using i) a rectified linear unit activation function, ii) a batch normalization function, or iii) a dropout function.

Optionally, the CNN model comprises at least first and second 1D convolutional layers, wherein the one or more processors are further configured to normalize an output of the first 1D convolutional layer, and send the normalized output of the first 1D convolutional layer to the second 1D convolutional layer.

Optionally, the CNN model further comprises a fully connected layer configured to receive input from the GAP layer.

Optionally, the CNN model outputs, in connection with each of a plurality of the DCA data sets, a confidence indicator indicative of a degree of confidence that the corresponding DCA data set represents a true positive or false positive designation of pause.

Optionally, the one or more processors are further configured to compare the confidence indicator to a detection threshold, wherein the information further comprises information concerning the valid subset of the DCA data sets that exceed the detection threshold.

Optionally, in response to an adjustment to the detection threshold, the one or more processors are further configured to present the information concerning the valid subset of the DCA data sets that exceed the adjusted detection threshold.

Optionally, the CNN model comprises five 1-dimensional (1D) convolutional layers, the GAP layer configured to receive outputs from each of the at least two 1D convolutional layers.

Optionally, the CNN model represents a model that is trained utilizing an augmented collection of DCA data sets, wherein the augmented collection of the DCA data sets includes reference DCA data sets from patients and synthetic DCA data sets that are generated based on the reference DCA data sets.

Optionally, the synthetic DCA data sets are generated using i) noise addition, ii) signal inversion, iii) magnification, iv) inserting one or more p waves during a pause interval at a previous R-R interval, v) stretching or shrinking a duration of a pause interval, or vi) two or more of noise addition, signal inversion, magnification, inserting one or more p waves during a pause interval at a previous R-R interval, or stretching or shrinking a duration of a pause interval.

Optionally, the information is indicative of a recommendation for adjustment to a sensing parameter or a therapy parameter associated with the IMD.

Optionally, the system further comprises the IMD, the IMD comprising a combination of subcutaneous electrodes configured to collect the CA signals, IMD memory configured to store program instructions, and one or more IMD processors configured to execute the program instructions to analyze the CA signals and based on the analysis declare candidate pause episodes and generate the DCA data sets including the corresponding CA signals. The system also comprises a transceiver configured to wirelessly transmit the DCA data sets to an external device.

Optionally, the system further comprises an external device that includes the memory and the one or more processors and a transceiver, the transceiver configured to wirelessly receive the DCA data sets from the IMD.

Optionally, the system further comprises a server that includes the memory and the one or more processors, the memory configured to store a collection of the DCA data sets, the one or more processors configured to apply the CNN model to the collection of the DCA data sets.

In accordance with embodiments herein, a computer implemented method to confirm device documented (DD) pause episodes comprises, under control of one or more processors configured with specific executable instructions, obtaining device classified arrhythmia (DCA) data sets generated by an implantable medical device (IMD) for corresponding candidate pause episodes declared by the IMD, the DCA data sets including cardiac activity (CA) signals for one or more beats sensed by the IMD. The method applies a convolutional neural network (CNN) model trained to detect pause episodes to the DCA data sets to identify a valid subset of the DCA data sets that correctly characterizes the corresponding CA signals. The method presents information concerning the valid subset of the DCA data sets.

Optionally, the method further comprises applying the CNN model to the DCA data sets to identify an invalid subset of the DCA data sets that incorrectly characterizes the corresponding CA signals, wherein the valid subset of the DCA data sets indicates a portion of the DCA data sets that indicates true pause, and the invalid subset of the DCA data sets indicates a second portion of the DCA data sets that indicates false pause.

Optionally, the information is indicative of a recommendation for i) a change in a treatment for a patient associated with the IMD that will increase the DCA data sets identified as the valid subset of the DCA data sets, or ii) a change in a treatment associated with the IMD that will increase the DCA data sets identified as the valid subset of the DCA data sets.

Optionally, the method further comprises applying the CNN model to the DCA data sets to identify an invalid subset of the DCA data sets that incorrectly characterizes the corresponding CA signals. In response to the change in the treatment for the patient or the IMD, the method further comprises obtaining second DCA data sets generated by the IMD, applying the CNN model to the second DCA data sets to identify a second valid subset of the DCA data sets that correctly characterizes the corresponding CA signals and to identify a second invalid subset of the DCA data sets that incorrectly characterizes the corresponding CA signals, and confirming that the second invalid subset of the DCA data sets has fewer invalid candidate pause episodes compared to the invalid subset of the DCA data sets.

Optionally, the method further comprises tuning the CNN model by inputting, into a layer of the CNN model, at least one of i) age-related feature, ii) gender, iii) body mass index, iv) ethnicity, v) medical history, vi) medication usage, vii) lifestyle factor, or viii) family history.

Optionally, wherein the CNN model comprises at least two layers, the method further comprises freezing one of the at least two layers, and updating, using additional DCA data sets, at least one layer of the CNN model that is not frozen.

In accordance with embodiments herein, in Example 1, a system (200) for declaring pause in cardiac activity comprises: memory (288) to store specific executable instructions and a convolutional neural network (CNN) model trained to detect pause episodes, the CNN model comprising a global average pooling (GAP) layer; one or more processors (220) configured to execute the specific executable instructions to: obtain device classified arrhythmia (DCA) data sets generated by an implantable medical device (IMD) (212) for corresponding candidate pause episodes declared by the IMD (212), the DCA data sets including cardiac activity (CA) signals for one or more beats sensed by the IMD (212); and apply the CNN model to the DCA data sets to identify a valid subset of the DCA data sets that correctly characterizes the corresponding CA signals; and a display configured to present information concerning the valid subset of the DCA data sets.

Optionally, in Example 2, in the system of Example 1, wherein the one or more processors (220) are further configured to apply the CNN model to the DCA data sets to identify an invalid subset of the DCA data sets that incorrectly characterizes the corresponding CA signals, wherein the valid subset of the DCA data sets indicates a portion of the DCA data sets that indicates true pause, and the invalid subset of the DCA data sets indicates a second portion of the DCA data sets that indicates false pause.

Optionally, in Example 3, in the system of Example 1 or 2, wherein the information is indicative of a recommendation for i) a change in a treatment for a patient (208) associated with the IMD (212) that will increase the DCA data sets identified as the valid subset of the DCA data sets, or ii) a change in a treatment associated with the IMD (212) that will increase the DCA data sets identified as the valid subset of the DCA data sets.

Optionally, in Example 4, in the system of Example 3, wherein the one or more processors (220) are further configured to apply the CNN model to the DCA data sets to identify an invalid subset of the DCA data sets that incorrectly characterizes the corresponding CA signals, wherein in response to the change in the treatment for the patient (208) or the IMD (212), the one or more processors (220) are further configured to: obtain second DCA data sets generated by the IMD (212); apply the CNN model to the second DCA data sets to identify a second invalid subset of the DCA data sets that incorrectly characterizes the corresponding CA signals; and confirm that the second invalid subset of the DCA data sets has fewer invalid candidate pause episodes compared to the invalid subset of the DCA data sets.

Optionally, in Example 5, in the system of any one of Examples 1 to 4, wherein the CNN model comprises at least two 1-dimensional (1D) convolutional layers, the GAP layer configured to receive outputs from each of the at least two 1D convolutional layers, wherein the one or more processors (220) are further configured to process an output of a first 1D convolutional layer using i) a rectified linear unit activation function, ii) a batch normalization function, or iii) a dropout function.

Optionally, in Example 6, in the system of any one of Examples 1 to 4, wherein the CNN model comprises at least first and second 1-dimensional (1D) convolutional layers, wherein the one or more processors (220) are further configured to: normalize an output of the first 1D convolutional layer; and send the normalized output of the first 1D convolutional layer to the second 1D convolutional layer.

Optionally, in Example 7, in the system of any one of Examples 1 to 6, wherein the CNN model further comprises a fully connected layer configured to receive input from the GAP layer.

Optionally, in Example 8, in the system of any one of Examples 1 to 7, wherein the CNN model outputs, in connection with each of a plurality of the DCA data sets, a confidence indicator indicative of a degree of confidence that the corresponding DCA data set represents a true positive or false positive designation of pause.

Optionally, in Example 9, in the system of Example 8, wherein the one or more processors (220) are further configured to compare the confidence indicator to a detection threshold, wherein the information further comprises information concerning the valid subset of the DCA data sets that exceed the detection threshold.

Optionally, in Example 10, in the system of Example 9, wherein in response to an adjustment to the detection threshold, the one or more processors (220) are further configured to present the information concerning the valid subset of the DCA data sets that exceed the adjusted detection threshold.

Optionally, in Example 11, in the system of any one of Examples 1 to 10, wherein the CNN model comprises five 1-dimensional (1D) convolutional layers, the GAP layer configured to receive outputs from each of the at least two 1D convolutional layers.

Optionally, in Example 12, in the system of any one of Examples 1 to 11, wherein the CNN model represents a model that is trained utilizing an augmented collection of DCA data sets, wherein the augmented collection of the DCA data sets includes reference DCA data sets from patients and synthetic DCA data sets that are generated based on the reference DCA data sets, wherein the synthetic DCA data sets are generated using i) noise addition, ii) signal inversion, iii) magnification, iv) inserting one or more p waves during a pause interval at a previous R-R interval, v) stretching or shrinking a duration of a pause interval, or vi) two or more of noise addition, signal inversion, magnification, inserting one or more p waves during a pause interval at a previous R-R interval, or stretching or shrinking a duration of a pause interval.

Optionally, in Example 13, in the system of any one of Examples 1 to 12, wherein the information is indicative of a recommendation for adjustment to a sensing parameter or a therapy parameter associated with the IMD (212).

Optionally, in Example 14, in the system of any one of Examples 1 to 13, further comprising the IMD (212), the IMD (212) comprising: a combination of subcutaneous electrodes configured to collect the CA signals (114, 126); IMD memory (288) configured to store program instructions; and one or more IMD processors (220) configured to execute the program instructions to: analyze the CA signals and based on the analysis declare candidate pause episodes; and generate the DCA data sets including the corresponding CA signals; and a transceiver (264) configured to wirelessly transmit the DCA data sets to an external device (214).

In accordance with embodiments herein, in Example 15, a computer implemented method to confirm device documented (DD) pause episodes, comprising: under control of one or more processors configured with specific executable instructions, obtaining device classified arrhythmia (DCA) data sets generated by an implantable medical device (IMD) for corresponding candidate pause episodes declared by the IMD, the DCA data sets including cardiac activity (CA) signals for one or more beats sensed by the IMD; applying a convolutional neural network (CNN) model trained to detect pause episodes to the DCA data sets to identify a valid subset of the DCA data sets that correctly characterizes the corresponding CA signals; and presenting information concerning the valid subset of the DCA data sets.

## BRIEF DESCRIPTION OF THE DRAWINGS

Figure 1A illustrates an implantable cardiac monitor (ICM) intended for subcutaneous implantation at a site near the heart in accordance with embodiments herein.

Figure 1B shows a block diagram of the ICM formed in accordance with embodiments herein.

Figure 2A illustrates a simplified block diagram of a system including an IMD in accordance with embodiments herein.

Figure 2B shows a block diagram of the system of Figure 2A in accordance with embodiments herein.

Figure 3 shows a high-level overview of a system formed in accordance with embodiments herein.

Figure 4A illustrates a summary of an example convolutional neural network (CNN) model utilized in accordance with embodiments herein.

Figure 4B illustrates three different electrogram (EGM) samples from different ICM devices including pause episodes in accordance with embodiments herein.

Figure 4C shows another subcutaneous EGM (SEGM) sample including a pause episode in accordance with embodiments herein.

Figure 4D shows examples of a fully connected layer and a global average pooling layer in accordance with embodiments herein.

Figure 4E illustrates a table of exemplary hyperparameters of interest in accordance with embodiments herein.

Figure 4F shows example equations that may be used to calculate a plurality of metrics in accordance with embodiments herein.

Figure 5 illustrates a process for training/building a CNN model to analyze DCA data sets, relative to an arrhythmia of interest such as pause, in accordance with embodiments herein.

Figure 6A illustrates a process for discriminating between valid and invalid device classified pause episodes in accordance with embodiments herein.

Figure 6B illustrates a process for using the CNN model to determine and/or implement treatment for a patient in accordance with embodiments herein.

Figure 7 illustrates a distributed processing system in accordance with embodiments herein.

Figure 8 illustrates a system level diagram indicating potential devices and networks that utilize the methods and systems herein.

DETAILED DESCRIPTION

The terms "cardiac activity signal", "cardiac activity signals", "CA signal" and "CA signals" (collectively "CA signals") are used interchangeably throughout and shall mean an analog or digital electrical signal recorded by two or more electrodes positioned transvenous, subcutaneous or cutaneous, where the electrical signals are indicative of cardiac electrical activity. The cardiac activity may be normal/healthy or abnormal/arrhythmic. Non-limiting examples of CA signals include ECG signals collected by cutaneous electrodes, and EGM signals collected by transvenous electrodes and/or non-transvenous, subcutaneous electrodes (e.g., SEGM). EGM and SEGM are used interchangeably herein.

The term "sensing vector" shall refer to a path extending between two or more physical, actual electrodes that operate as sensing sites.

The term "subcutaneous" shall be below the skin surface but not within the heart and not transvenous.

The terms "device classified arrhythmia data set" and "DCA data set" are used interchangeably and shall mean a data set that includes i) CA signals collected in response to a determination by an IMD that the CA signals are indicative of an arrhythmia of interest and ii) one or more device documented markers related to one or more features of interest in the CA signals that in whole or in part were utilized by the IMD in connection with the determination of the arrhythmia of interest. Additionally or alternatively, the DCA data set shall include the CA signals collected in response to a determination by an IMD that the CA signals are indicative of an arrhythmia of interest, but not include device documented markers. In some cases, the arrhythmia of interest is a pause episode or event.

The terms "device classified normal sinus data set" and "DCNS data set" are used interchangeably and shall mean a data set that includes i) CA signals collected in response to a determination by an IMD that the CA signals are indicative of a normal sinus rhythm and ii) one or more device documented markers related to one or more features of interest in the CA signals that in whole or in part were utilized by the IMD in connection with the determination of the normal sinus rhythm. Additionally or alternatively, the DCNS data set shall include the CA signals collected in response to a determination by an IMD that the CA signals are indicative of a normal sinus rhythm, but not include device documented markers.

The term "device documented marker" refers to markers that are generated by an IMD to characterize one or more features of interest within respective CA signals. Markers may be declared based on numerous criteria, such as signal processing, feature detection and arrhythmia detection software and hardware within and/or operating on the implantable cardiac monitor and/or implantable medical device.

The term "marker" shall mean data and/or information identified from CA signals that may be presented as graphical and/or numeric indicia indicative of one or more features within the CA signals and/or indicative of one or more episodes exhibited by the cardiac events. Markers may be superimposed upon CA signals or presented proximate to, and temporally aligned with, CA signals. Non-limiting examples of markers may include R-wave markers, noise markers, activity markers, interval markers, refractory markers, P-wave markers, T-wave markers, PVC markers, sinus rhythm markers, AF markers, pause markers, and other arrhythmia markers. As a further nonlimiting example, basic event markers may include "AF entry" to indicate a beginning of an AF event, "in AF" to indicate that AF is ongoing, "AF exit" to indicate that AF has terminated, "pause entry" to indicate a beginning of a pause event, "in pause" to indicate that pause is ongoing, "pause exit" to indicate that pause has terminated, "T" to indicate a tachycardia beat, "B" to indicate a bradycardia beat, "A" to indicate an asystole beat, "VS" to indicate a regular sinus beat, "Tachy" to indicate a tachycardia episode, "Brady" to indicate a Bradycardia episode, "Asystole" to indicate an asystole episode, "Patient activated" to indicate a patient activated episode. An activity marker may indicate activity detected by activity sensor during the CA signal. Noise markers may indicate entry/start, ongoing, recovery and exit/stop of noise. Markers may be presented as symbols, dashed lines, numeric values, thickened portions of a waveform, and the like. Markers may represent events, intervals, refractory periods, ICM activity, and other algorithm related activity. For example, interval markers, such as the R-R interval and/or pause interval (e.g., pause period), may include a numeric value indicating the duration of the interval. The AF markers indicate atrial fibrillation rhythm.

The term "synthetic DCA data sets" shall mean data sets that include artificially generated or computer-generated CA signals, where the CA signals and DD markers are based on actual DCA data sets collected from a patient, but where the CA signals are not collected from an actual patient.

The terms "beat" and "cardiac event" are used interchangeably and shall include both normal or abnormal events.

The terms "normal" and "sinus" are used to refer to events, features, and characteristics of, or appropriate to, a heart's healthy or normal functioning.

The terms "abnormal", "arrhythmic", or "pause" are used to refer to events, features, and characteristics of, or appropriate to, a un-healthy or abnormal functioning of the heart.

The term "machine learning" shall mean an artificial intelligence algorithm that learns from various automatic or manual inputs, such as features of interest, prior device classified arrhythmias, observations and/or data. The machine learning algorithm is adjusted over multiple iterations based on the features of interest, prior device classified arrhythmias, observations and/or data. For example, the machine learning algorithm is adjusted by supervised learning, unsupervised learning, and/or reinforcement learning. Non-limiting examples of machine learning algorithms are a convolutional neural network (CNN), gradient boosting random forest, decision tree, K-means, deep learning, artificial neural network, and/or the like. The machine learning model may include a CNN architecture. It is recognized that the network architecture may differ and/or other types of machine learning models may be utilized. As non-limiting examples, the architecture may comprise N network layers, each with M sub-layers followed by pooling and normalization. The architecture components may include: 1-dimensional convolutional layers ("Conv1D"), rectified linear unit ("relu") activation functions, batch normalization ("BN"), etc. The network output may be a continuous value between 0 and 1, where values close to zero indicate high confidence that the CA signals indicate a false positive of pause, and values close to 1 indicate high confidence that the CA signals indicate a true positive of pause.

The term "real-time" refers to a time frame substantially contemporaneous with occurrence of a normal or abnormal episode or an event of interest. For example, a real-time process or operation would occur during or immediately after (e.g., within minutes or seconds after) an arrhythmia episode such as pause, upon the availability of data, and the like.

The term "obtain", as used in connection with data, signals, information and the like, includes at least one of i) accessing memory of an IMD, ICM, external device or remote server where the data, signals, information, etc., are stored, ii) receiving the data, signals, information, etc., over a wireless communications link between the ICM or IMD and a local external device, iii) receiving the data, signals, information, etc., at a remote server over a network connection and/or iv) sensing or collecting signals (e.g., CA signals, impedance signals, etc.) between a combination of electrodes provided on or coupled to the ICM or IMD. An obtaining operation, when from the perspective of an ICM or IMD, may include sensing new signals in real-time, and/or accessing memory to read stored data, signals, information, etc., from memory within the ICM or IMD. The obtaining operation, when from the perspective of a local external device, includes receiving the data, signals, information, etc., at a transceiver of the local external device where the data, signals, information, etc., are transmitted from an ICM and/or a remote server. The obtaining operation may be from the perspective of a remote server, such as when receiving the data, signals, information, etc., at a network interface from a local external device and/or directly from an ICM. The remote server may also obtain the data, signals, information, etc., from local memory and/or from other memory, such as within a cloud storage environment and/or from the memory of a workstation or clinician external programmer.

The terms "recommend" and "recommendation" shall mean a proposed action that is taken by the patient, medical personnel and/or a device (e.g., an IMD, local external device, remote server, programmer, smart phone) to alter a treatment and/or maintain a current course of action (e.g., maintain a treatment).

The term "treatment' shall mean one or more of the following: Non-limiting examples of treatment include instructing the medical professional to change a patient's medication or dose, edit a diagnosis, clear alerts, change one or more parameters within the IMD (e.g., sensing parameters, shock parameters, threshold(s) associated with detecting true pause and false pause, adjustments based on a confidence indicator indicative of a degree of confidence that a corresponding DCA data set represents a true pause or false pause, and/or pacing parameters). The examples of treatment may also include an instruction to change, maintain, add or stop a therapy delivered by an active IMD, such as a pacing therapy, an ATP pacing therapy, a neural stimulation therapy, mechanical circulatory support and the like. The examples of treatment may further include dispatching an ambulance to the patient's location, instructing the patient to immediately go to a hospital, instructing the patient to schedule an appointment, instructing the patient to change a prescription, instructing the patient to undergo additional examinations (e.g., diagnostic imaging examinations, exploratory surgery and the like), instructing the patient to alter medication, diet, behavior, activity). In some cases, treatments may be automatically implemented, such as automatically adjusting a sensitivity level within a predetermined range, automatically adjusting a threshold level within a predetermined range, etc. A treatment can also determine how urgent/important the detected event is based on whether the patient has been previously diagnosed and is currently being treated, or whether this is a new diagnosis and start of therapy based on additional status inputs from the providers.

In accordance with embodiments herein, methods and systems train and utilize a convolutional neural network (CNN) model, with automatic classification of pause episodes, to determine whether candidate arrhythmias (e.g., pause), declared and classified by an IMD from EGMs and/or subcutaneous EGMs (SEGMs), are true or false positives. The CNN model provides the highest sensitivity and specificity in order to reduce false episodes as much as possible, while maintaining true episodes.

The CNN model can be trained using data sets including adjudicated true pause episodes and datasets that do not include pause episodes. Additional training data can be generated by augmenting the data sets using one or more augmentation techniques, such as inversion, magnification, noise addition, inserting one or more p waves during a pause interval at a previous R-R interval, and/or stretching or shrinking a duration of a pause interval. More than one CNN model can be trained to detect pause events, such as by using different training data, different augmented training data, etc.

The trained CNN model can evaluate patient data acquired by an implantable device. The trained CNN model determines the probability of the episode recorded by the implantable device being a true pause or a false pause event. The true pause events can be sent to a clinician, thus reducing the burden for reviewing all possible pause events.

In addition, the trained CNN model can be used to adjust therapy parameters to improve treatment of the patient. Therapy parameters can include, but are not limited to, detection thresholds, sensitivity levels, therapy settings, and the like.

The trained CNN model can be personalized for one or more patient, and can be further modified by transfer learning to identify other related events within patient data. A clinician can tune the CNN model based on particular patient information.

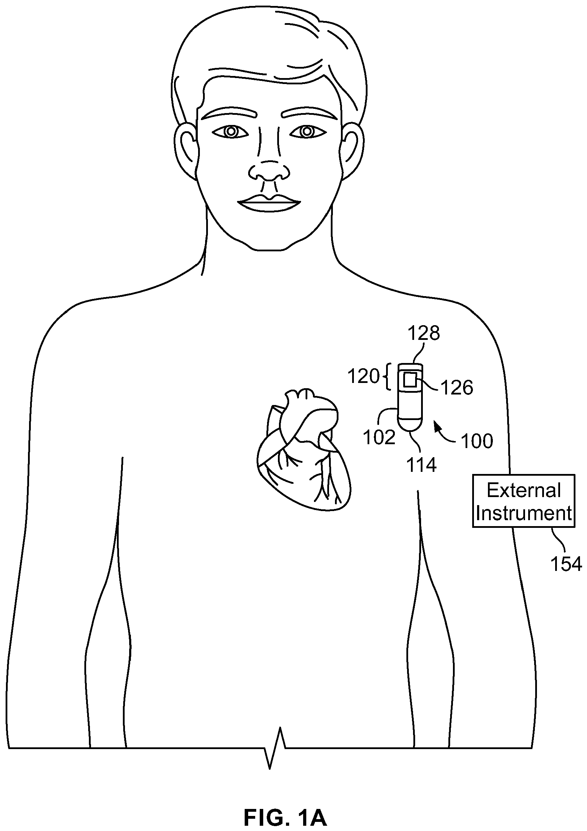

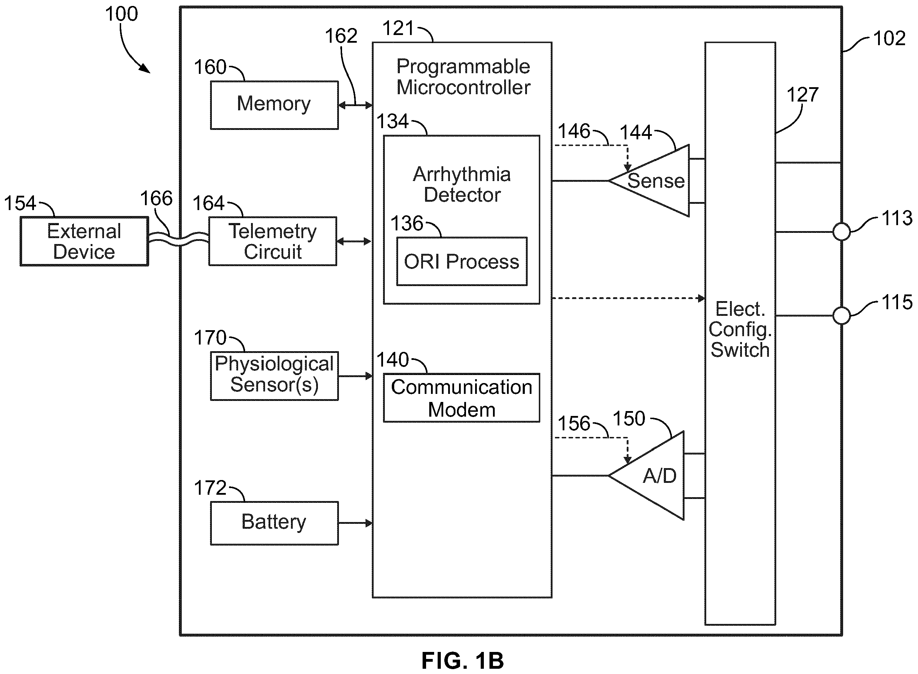

Figure 1 illustrates an implantable medical device (IMD) 100 intended for subcutaneous implantation at a site near the heart. In some cases, the IMD 100 can be an ICM that is subcutaneously implanted at a site near the heart. It should be understood that the embodiments herein apply equally to other implantable medical devices, such as leadless monitoring and/or therapy devices, cardiac monitoring device, pacemaker, cardioverter, cardiac rhythm management device, defibrillator, leadless monitoring device, leadless pacemaker, and/or leadless implantable medical device (LIMD), which are implanted near and/or within the heart, and are configured to receive near-field and/or far-field cardiac CA signals. The IMD 100 includes a pair of spaced-apart sense electrodes 114, 126 positioned with respect to a housing 102. The sense electrodes 114, 126 provide for detection of far field electrogram signals. Numerous configurations of electrode arrangements are possible. For example, the electrode 114 may be located on a distal end of the IMD 100, while the electrode 126 is located on a proximal side of the IMD 100. Additionally or alternatively, electrodes 126 may be located on opposite sides of the IMD 100, opposite ends or elsewhere. The distal electrode 114 may be formed as part of the housing 102, for example, by coating all but a portion of the housing with a nonconductive material such that the uncoated portion forms the electrode 114. In this case, the electrode 126 may be electrically isolated from the housing 102 electrode by placing it on a component separate from the housing 102, such as the header 120. Optionally, the header 120 may be formed as an integral portion of the housing 102. The header 120 includes an antenna 128 and the electrode 126. The antenna 128 is configured to wirelessly communicate with an external device 154 in accordance with one or more predetermined wireless protocols (e.g., Bluetooth, Bluetooth low energy, Wi-Fi, etc.). The housing 102 includes various other components such as: sense electronics for receiving signals from the electrodes, a microprocessor for processing the signals in accordance with algorithms, such as the arrhythmia and pause algorithm(s) described herein, a loop memory for temporary storage of CA data, a device memory for long-term storage of CA data upon certain triggering events, such as pause detection, sensors for detecting patient activity and a battery for powering components. Although Figure 1 is directed to an ICM, the embodiments disclosed herein apply to other implantable medical devices (IMD), and the terms IMD and ICM are used interchangeably herein.

In at least some embodiments, the IMD 100 is configured to be placed subcutaneously utilizing a minimally invasive approach. Subcutaneous electrodes are provided on the housing 102 to simplify the implant procedure and eliminate a need for a transvenous lead system. The sensing electrodes may be located on opposite sides of the device and designed to provide robust episode detection through consistent contact at a sensor-tissue interface. The IMD 100 may be configured to be activated by the patient or automatically activated, in connection with recording EGM/SEGM signals.

The IMD 100 senses far field, subcutaneous CA signals, processes the CA signals to detect arrhythmias such as pause events, and if pause is detected, automatically records the CA signals in memory for subsequent transmission to an external device. The CA signal processing and pause detection is provided for, at least in part, by algorithms embodied in or implemented by the microprocessor. The IMD 100 includes one or more processors and memory that stores program instructions directing the processors to implement pause detection and analyze cardiac activity signals collected over one or more sensing channels.

Figure 1B shows a block diagram of the IMD 100 formed in accordance with embodiments herein. The IMD 100 may be implemented to monitor ventricular activity alone, or both ventricular and atrial activity through sensing circuit. The IMD 100 has a housing 102 to hold the electronic/computing components. The housing 102 (which is often referred to as the "can", "case", "encasing", or "case electrode") may be programmably selected to act as an electrode for certain sensing modes. Housing 102 further includes a connector (not shown) with at least one terminal 113 and optionally additional terminals 115. The terminals 113, 115 may be coupled to sensing electrodes that are provided upon or immediately adjacent the housing 102. Optionally, more than two terminals 113, 115 may be provided in order to support more than two sensing electrodes, such as for a bipolar sensing scheme that uses the housing 102 as a reference electrode. Additionally or alternatively, the terminals 113, 115 may be connected to one or more leads having one or more electrodes provided thereon, where the electrodes are located in various locations about the heart. The type and location of each electrode may vary.

The IMD 100 includes a programmable microcontroller 121 that controls various operations of the IMD 100, including cardiac monitoring. Microcontroller 121 includes a microprocessor (or equivalent control circuitry), RAM and/or ROM memory, logic and timing circuitry, state machine circuitry, and I/O circuitry. The microcontroller 121 also performs the operations described herein in connection with collecting cardiac activity data and analyzing the cardiac activity data.

A switch 127 is optionally provided to allow selection of different electrode configurations under the control of the microcontroller 121. The electrode configuration switch 127 may include multiple switches for connecting the desired electrodes to the appropriate I/O circuits, thereby facilitating electrode programmability. The switch 127 is controlled by a control signal from the microcontroller 121. Optionally, the switch 127 may be omitted and the I/O circuits directly connected to the housing electrode 114 and a second electrode 126 (Figure 1A).

Microcontroller 121 includes an arrhythmia detector 134 that is configured to analyze cardiac activity signals to identify potential arrhythmia episodes (e.g., Tachycardias, Bradycardias, Asystole, Brady pause, pause, atrial fibrillation, etc.). By way of example, the arrhythmia detector 134 may implement arrhythmia detection algorithm(s) as described in U.S. Patent 8,135,456, entitled "Device and method for detecting atrial fibrillation" and issued on March 13, 2012, as described in U.S. Patent 11,559,241, entitled "Methods and systems for reducing false declarations of arrhythmias" and issued on January 24, 2023, and/or as described in U.S. Patent 11,633,141, entitled "Method and system to detect r-waves in cardiac arrhythmic patterns" and issued on April 25, 2023. Although not shown, the microcontroller 121 may further include other dedicated circuitry and/or firmware/software components that assist in monitoring various conditions of the patient's heart and managing pacing therapies.

The arrhythmia detector 134 of the microcontroller 121 includes an on-board R-R interval irregularity (ORI) process 136 that detects arrhythmia episodes, such as AF episodes using R-R interval irregularities. The ORI process 136 may be implemented as firmware, software and/or circuits. The ORI process 136 uses a hidden Markov Chains and Euclidian distance calculations of similarity to assess the transitionary behavior of one R-wave (RR) interval to another and compare the patient's RR interval transitions to the known RR interval transitions during atrial fibrillation (AF) and non-AF episodes obtained from the same patient and/or many patients.

The microcontroller 121 can include other arrhythmia determination circuitry/software/firmware (not shown) that is configured to analyze the CA signals to identify the existence of an arrhythmia, and more specifically pause. Additional circuitry/software/firmware may also analyze motion of the patient when determining pause.

The arrhythmia detector 134 analyzes sensed far field CA signals sensed along a sensing vector between a combination of subcutaneous electrodes for one or more beats. The arrhythmia detector 134 identifies one or more features of interest from the CA signals, and based on further analysis of the features of interest determines whether the CA signals are indicative of a normal sinus rhythm or an arrhythmia episode. When an arrhythmia episode is identified, the arrhythmia detector 134 generates one or more DD markers that are temporally aligned with corresponding features of interest in the CA signals. The arrhythmia detector 134 forms a DCA data set associated with the classified arrhythmia episode (e.g., pause episode) and stores the DCA data set in the memory 160 of the IMD 100. The arrhythmia detector 134 iteratively or periodically repeats the analysis of incoming far field CA signals to continuously add DCA data sets for respective arrhythmia episodes, thereby forming a collection of DCA data sets.

The IMD 100 is further equipped with a communication modem (modulator/demodulator) 140 to enable wireless communication. In one implementation, the communication modem 140 uses high frequency modulation, for example using RF, Bluetooth or Bluetooth Low Energy telemetry protocols. The signals are transmitted in a high frequency range and will travel through the body tissue in fluids without stimulating the heart or being felt by the patient. The communication modem 140 may be implemented in hardware as part of the microcontroller 121, or as software/firmware instructions programmed into and executed by the microcontroller 121. Alternatively, the modem 140 may reside separately from the microcontroller as a standalone component. The modem 140 facilitates data retrieval from a remote monitoring network. The modem 140 enables timely and accurate data transfer directly from the patient to an electronic device utilized by a physician or the patient.

The IMD 100 includes sensing circuit 144 selectively coupled to one or more electrodes that perform sensing operations, through the switch 127 to detect cardiac activity data indicative of cardiac activity. The sensing circuit 144 may include dedicated sense amplifiers, multiplexed amplifiers, or shared amplifiers. It may further employ one or more low power, precision amplifiers with programmable gain and/or automatic gain control, bandpass filtering, and threshold detection circuit to selectively sense the features of interest. In one embodiment, switch 127 may be used to determine the sensing polarity of the cardiac signal by selectively closing the appropriate switches.

The output of the sensing circuit 144 is connected to the microcontroller 121 which, in turn, determines when to store the cardiac activity data of CA signals (digitized by the A/D data acquisition system 150) in the memory 160. For example, the microcontroller 121 may only store the cardiac activity data (from the A/D data acquisition system 150) in the memory 160 when a potential arrhythmia episode is detected. The sensing circuit 144 receives a control signal 146 from the microcontroller 121 for purposes of controlling the gain, threshold, polarization charge removal circuitry (not shown), and the timing of any blocking circuitry (not shown) coupled to the inputs of the sensing circuit.

Optionally, the IMD 100 may include multiple sensing circuits, similar to sensing circuit 144, where each sensing circuit is coupled to two or more electrodes and controlled by the microcontroller 121 to sense electrical activity detected at the corresponding two or more electrodes. The sensing circuit 144 may operate in a unipolar sensing configuration or in a bipolar sensing configuration. Optionally, the sensing circuit 144 may be removed entirely and the microcontroller 121 perform the operations described herein based upon the CA signals from the A/D data acquisition system 150 directly coupled to the electrodes.

The IMD 100 further includes an analog-to-digital A/D data acquisition system (DAS) 150 coupled to one or more electrodes via the switch 127 to sample cardiac activity signals across any pair of desired electrodes. The data acquisition system 150 is configured to acquire cardiac electrogram (EGM) signals as CA signals, convert the raw analog data into digital data, and store the digital data as CA data for later processing and/or telemetric transmission to an external device 154 (e.g., a programmer, local transceiver, or a diagnostic system analyzer). The data acquisition system 150 is controlled by a control signal 156 from the microcontroller 121. The EGM signals may be utilized as the cardiac activity data that is analyzed for potential arrhythmia episodes such as pause. The ORI process 136 may be applied to signals from the sensing circuit 144 and/or the DAS 150.

By way of example, the external device 154 may represent a bedside monitor installed in a patient's home and utilized to communicate with the IMD 100 while the patient is at home, in bed or asleep. The external device 154 may be a programmer used in the clinic to interrogate the IMD 100, retrieve data and program detection criteria and other features. The external device 154 may be a handheld device (e.g., smartphone, tablet device, laptop computer, smartwatch and the like) that can be coupled over a network (e.g., the Internet) to a remote monitoring service, medical network and the like. The external device 154 facilitates access by physicians to patient data as well as permitting the physician to review real-time CA signals while collected by the IMD 100. In some cases the external device 154 may facilitate access by the patient, such as to view alerts, summaries, etc., associated with the patient data.

The microcontroller 121 is coupled to a memory 160 by a suitable data/address bus 162. The programmable operating parameters used by the microcontroller 121 are stored in memory 160 and used to customize the operation of the IMD 100 to suit the needs of a particular patient. Such operating parameters define, for example, detection rate thresholds, sensitivity, automatic features, pause detection criteria, AF detection criteria, activity sensing or other physiological sensors, and electrode polarity, etc.

In addition, the memory 160 stores the cardiac activity data, as well as the markers and other data content associated with detection of arrhythmia episodes. The operating parameters of the IMD 100 may be non-invasively programmed into the memory 160 through a telemetry circuit 164 in telemetric communication via communication link 166 with the external device 154. The telemetry circuit 164 allows intracardiac electrograms and status information relating to the operation of the IMD 100 (as contained in the microcontroller 121 or memory 160) to be sent to the external device 154 through the established communication link 166. In accordance with embodiments herein, the telemetry circuit 164 conveys the DCA data sets and other information related to arrhythmia episodes to an external device 154.

The IMD 100 may further include magnet detection circuitry (not shown), coupled to the microcontroller 121, to detect when a magnet is placed over the unit. A magnet may be used by a clinician to perform various test functions of the housing 102 and/or to signal the microcontroller 121 that the external device 154 is in place to receive or transmit data to the microcontroller 121 through the telemetry circuits 164.

The IMD 100 can further include one or more physiological sensors 170. Such sensors are commonly referred to (in the pacemaker arts) as "rate-responsive" or "exercise" sensors. The physiological sensor 170 may further be used to detect changes in the physiological condition of the heart, or diurnal changes in activity (e.g., detecting sleep and wake states). Signals generated by the physiological sensors 170 are passed to the microcontroller 121 for analysis and optional storage in the memory 160 in connection with the cardiac activity data, markers, episode information and the like. While shown as being included within the housing 102, the physiological sensor(s) 170 may be external to the housing 102, yet still be implanted within or carried by the patient. Examples of physiologic sensors might include sensors that, for example, detect motion, activity, temperature, sense respiration rate, pH of blood, ventricular gradient, activity, position/posture, minute ventilation (MV), and so forth.

A battery 172 provides operating power to all of the components in the IMD 100. The battery 172 is capable of operating at low current drains for long periods of time. The battery 172 also desirably has a predictable discharge characteristic so that elective replacement time can be detected. As one example, the housing 102 employs lithium/silver vanadium oxide batteries. The battery 172 may afford various periods of longevity (e.g., three years or more of device monitoring). In alternate embodiments, the battery 172 could be rechargeable. See for example, U.S. Patent Number 7,294,108, Cardiac event micro-recorder and method for implanting same.

The IMD 100 provides a simple to configure data storage option to enable physicians to prioritize data based on individual patient conditions, to capture significant events and reduce risk that unexpected events are missed. The IMD 100 may be programmable for pre- and post-trigger event storage. For example, the IMD 100 may be automatically activated to store 10-120 seconds of CA data prior to an event of interest and/or to store 10-120 seconds of post CA data. Optionally, the IMD 100 may afford patient triggered activation in which pre-event CA data is stored, as well as post event CA data (e.g., pre-event storage of 1-15 minutes and post-event storage of 1-15 minutes). Optionally, the IMD 100 may afford manual (patient triggered) or automatic activation for CA data. Optionally, the IMD 100 may afford additional programming options (e.g., asystole duration, bradycardia rate, tachycardia rate, tachycardia cycle count). The amount of CA data storage may vary based upon the size of the memory 160.

The IMD 100 may provide comprehensive safe diagnostic data reports including a summary of heart rate, in order to assist physicians in diagnosis and treatment of patient conditions. By way of example, reports may include episode diagnostics for auto trigger events, episode duration, episode count, episode date/time stamp and heart rate histograms. The IMD 100 may be configured to be relatively small (e.g., between 2-10 cc in volume) which may, among other things, reduce risk of infection during implant procedure, afford the use of a small incision, afford the use of a smaller subcutaneous pocket and the like. The small footprint may also reduce implant time and introduce less change in body image for patients.

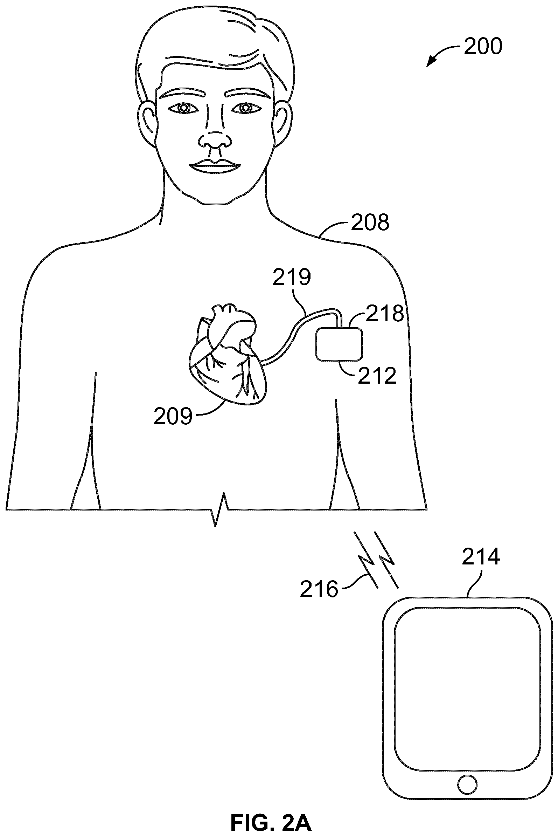

Figure 2A illustrates a simplified block diagram of a system 200 operated in accordance with embodiments herein. The system 200 includes an IMD 212 and one or more external devices or instruments. A single external device 214 is illustrated. The IMD 212 and the external device 214 are configured to communicate with one another wirelessly over a wireless communication link 216. In an embodiment, the external device 214 receives communications from a data processing system located remote from the external device 214, and transmits communications, including physiological data acquired from patient 208 such as DCA data sets and information identifying the IMD 212 and the type of data acquired by the IMD 212, to the data processing system.

The IMD 212 is implanted within a patient 208 at a site near the heart 209. The IMD 212 may be a cardiac pacemaker, an implantable cardiac monitoring device (ICM), a defibrillator, an ICM coupled with a pacemaker, or the like. The IMD 212 is intended for implantation within a subcutaneous pocket of the patient. The IMD 212 may be configured to sense cardiac signals to monitor cardiac activity over time. In some embodiments, the IMD 212 may also be configured to deliver stimulation therapy to the heart 209. For example, the IMD 212 may be a dual-chamber stimulation device capable of treating both fast and slow arrhythmias with stimulation therapy, including cardioversion, defibrillation, and pacing stimulation, as well as being capable of detecting heart failure, evaluating its severity, tracking the progression thereof, and controlling the delivery of therapy and warnings in response thereto.

The IMD 212 in the illustrated embodiment includes a body or housing 218 that is connected to at least one lead 219. A single lead 219 is shown in Figure 2A, but the IMD 212 may include multiple leads in another embodiment. The lead 219 extends from the housing 218 to the heart 209, such that the distal end is in contact with patient tissue surrounding the heart 209. The lead 219 includes one or more electrodes that may measure cardiac signals of the heart 209 and deliver stimulation therapy to the heart 209. In an embodiment, a single electrode may emit a stimulation pulse in a stimulation mode, and then may quickly switch to a monitoring mode to detect cardiac signals following the stimulation pulse.

Although the IMD 212 in the illustrated embodiment includes a lead 219, one or more of the embodiments described herein utilize a leadless IMD that does not include any lead. For example, the IMD 212 may be a leadless pacemaker, a leadless cardiac monitoring device (ICM), or the like. In general, the IMD 212 may represent a cardiac monitoring device, pacemaker, cardioverter, cardiac rhythm management device, defibrillator, neurostimulator, leadless monitoring device, leadless pacemaker, and the like.

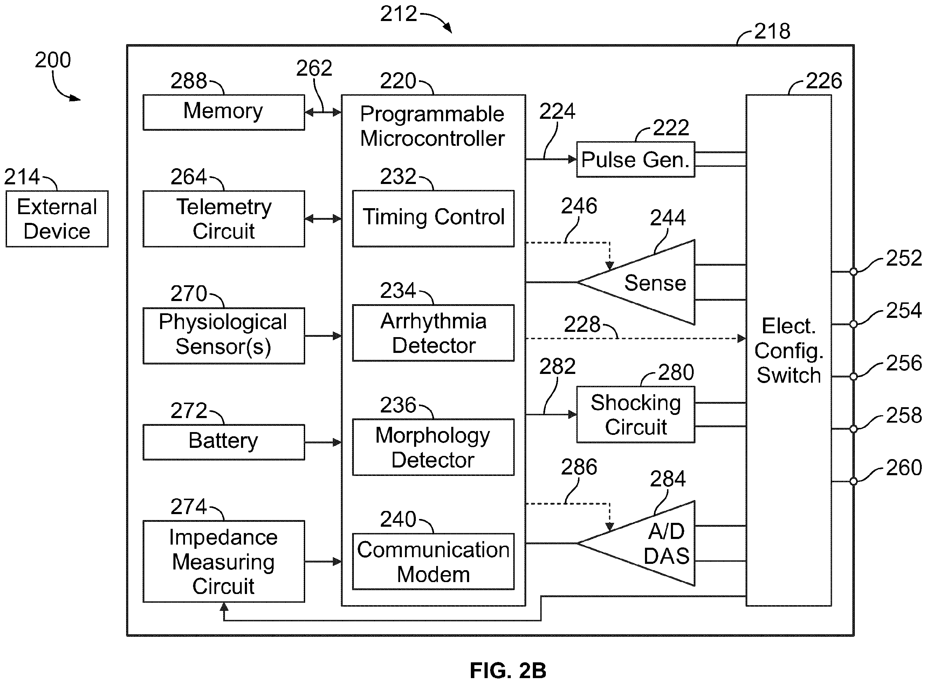

The housing 218 may contain a battery, pulse generation circuitry, communication circuitry, a data storage device (e.g., memory), and/or control circuitry including one or more processors. The control circuitry is for receiving and analyzing EGM signals from the electrodes. The control circuitry may include at least one processor for processing the EGM signals in accordance with algorithms to make determinations about the state of the heart 209. The memory provides storage for the cardiac signals and programmed instructions for the control circuitry that provide a programming session. The battery powers the circuitry within the housing 218. For example, the battery powers the pulse generation circuitry to generate stimulation pulses and powers the communication circuitry to communicate with an external device 214. The control circuitry may generate messages to be communicated via the communication circuitry to the external device 214. The messages may include the EGM signals and/or data generated based on the EGM signals. The control circuitry is also configured to analyze messages, received via the communication circuit from the external device 214, that include programming packages for updating the operating configuration, including settings, parameters, behavior, and the like, of the IMD 212. Exemplary structure for the IMD 212 is discussed and illustrated below in connection with Figure 2B.

The external device 214 may represent a computing device that is accessible or possessable by the patient or a clinician, such as a tablet computer, a smartphone, a wearable device, laptop computer, a desktop computer, a bedside monitor installed in a patient's home, or the like. The external device 214 alternatively may be a programmer device used in the clinic by a clinician. The external device may be one of the devices listed above that is communicatively connected to a second external device that represents the source of the programming package intended to update the operating configuration of the IMD 212. For example, the second external device may be a server or another computing device that is communicatively connected to the first external device 214 via a network connection (e.g., the Internet). In general, the external device 214 facilitates access by physicians to patient data as well as permits the physician to review real-time EGM signals and update the operating configuration of the IMD 212 without the clinician being near the patient, such as to facilitate a change in treatment accomplished by the IMD 212.

Figure 2B shows a block diagram of the system 200 in accordance with embodiments herein. The housing 218 or case of the IMD 212 holds the electronic and/or computing components. The housing 218 further includes a connector (not shown) with at least one terminal 252 and optionally additional terminals 254, 256, 258, 260. The terminals may be connected to electrodes that are located in various locations within and about the heart, such as on the lead 219 (shown in Figure 2A). The electrodes may include various combinations of ring, tip, coil, shocking electrodes, and the like.

The IMD 212 includes a programmable microcontroller 220 that controls various operations of the IMD 212, including cardiac monitoring and stimulation therapy. Microcontroller 220 includes one or more processors (e.g., a microprocessor or equivalent control circuitry), RAM and/or ROM memory, logic and timing circuitry, state machine circuitry, and I/O circuitry. Microcontroller 220 includes an arrhythmia detector 234 that is configured to detect cardiac activity data to identify potential atrial fibrillation (AF) episodes and pause as well as other arrhythmias (e.g., Tachycardias, Bradycardias, Asystole, etc.).

An electrode configuration switch 226 is optionally provided to allow selection of different electrode configurations under the control of the microcontroller 220. The electrode configuration switch 226 may include multiple switches for connecting the desired electrodes to the appropriate I/O circuits, thereby facilitating electrode programmability. The switch 226 is controlled by a control signal 228 from the microcontroller 220. Optionally, the switch 226 may be omitted and the I/O circuits directly connected to a housing electrode.

The IMD 212 may include a chamber pulse generator 222 that generates stimulation pulses for connecting the desired electrodes to the appropriate I/O circuits, thereby facilitating electrode programmability. The pulse generator 222 is controlled by the microcontroller 220 via control signals 224. The IMD 212 includes a sensing circuit 244 selectively coupled to one or more electrodes that perform sensing operations through the switch 226 to detect cardiac activity. The sensing circuit 244 may include dedicated sense amplifiers, multiplexed amplifiers, or shared amplifiers. The sensing circuit 244 may operate in a unipolar sensing configuration or a bipolar sensing configuration. The output of the sensing circuit 244 is connected to the microcontroller 220 which, in turn, triggers, or inhibits the pulse generator 222 in response to the absence or presence of cardiac activity. The sensing circuit 244 receives a control signal 246 from the microcontroller 220 for purposes of controlling the gain, threshold, polarization, and timing of any blocking circuitry (not shown) coupled to the sensing circuit.

The IMD 212 further includes an analog-to-digital A/D data acquisition system (DAS) 284 coupled to one or more electrodes via the switch 226 to sample cardiac signals across any pair of desired electrodes. The A/D DAS 284 is controlled by a control signal 286 from the microcontroller 220.

The IMD 212 is further equipped with a communication circuit or modem (modulator/demodulator) 240 to enable wireless communication. The modem 240 enables timely and accurate data transfer directly from the patient to the external device 214, and vice-versa. For example, the communication modem 240 is configured to establish the communication link 216 (Figure 2A) with the external device 214. In an embodiment, the communication modem 240 can receive a programming package from the external device 214 that conveys treatment settings for updating, changing, varying, etc. In addition to remote programming of the IMD 212, the wireless communication link 216 with the external device 214 facilitates access by physicians and/or patients to patient data such as DCA data sets generated by the IMD 212.

The communication modem 240 may utilize radio frequency (RF), Bluetooth, or Bluetooth Low Energy telemetry protocols. The signals are transmitted in a high frequency range and will travel through the body tissue in fluids without stimulating the heart or being felt by the patient. The communication modem 240 may be implemented in hardware as part of the microcontroller 220, or as software/firmware instructions programmed into and executed by the microcontroller 220. Alternatively, the modem 240 may reside separately from the microcontroller 220 as a standalone hardware component.

The microcontroller 220 is coupled to a non-transitory data storage device, referred to herein as memory device 288, by a suitable data/address bus 262. The memory device 288 stores programmable operating parameters used by the microcontroller 220 and/or data associated with the detection and determination of arrhythmias. The memory 288 can store a CNN model that has been trained to detect pause episodes. In an embodiment, the memory device 288 also stores the current programmed settings, along with any and all changes, modifications, updates, or the like previously made to the programmed settings.

The IMD 212 optionally includes one or more physiologic sensors 270 that adjust pacing stimulation rates, detect changes in cardiac output, changes in the physiological condition of the heart, and/or diurnal changes in activity (e.g.,

detecting sleep and wake states). Examples of physiological sensors 270 might include sensors that, for example, sense respiration rate, pH of blood, ventricular gradient, activity, body movement, position/posture, minute ventilation (MV), and/or the like.

The battery 272 provides operating power to all of the components in the IMD 212. The battery 272 is capable of operating at low current drains for long periods of time, and is capable of providing high-current pulses (for capacitor charging) when the patient requires a shock pulse (e.g., in excess of 2 A, at voltages above 2 V, for periods of 10 seconds or more).

The IMD 212 further includes an impedance measuring circuit 274, which can be used for many things, including sensing respiration phase. The IMD 212 may be further equipped with a telemetry circuit 264 that can selectively communicate with an external device, such as the device 214, when connected via a physical (e.g., wired) communication link or a wireless link. The IMD 212 includes a shocking circuit 280 controlled by control signals 282 generated by the microcontroller 220. The shocking circuit 280 generates shocking pulses of low (e.g., up to 0.5 joules), moderate (e.g., 0.5-10 joules), or high energy (e.g., 11 to 40 joules), as controlled by the microcontroller 220.

The microcontroller 220 may include other dedicated circuitry and/or firmware/software components, such as a timing control (module) 232 and a morphology detector (module) 236. The timing control 232 is used to control various timing parameters, such as stimulation pulses (e.g., pacing rate, atria-ventricular (AV) delay, atrial interconduction (A-A) delay, ventricular interconduction (V-V) delay, etc.) as well as to keep track of the timing of RR-intervals, refractory periods, blanking intervals, noise detection windows, evoked response windows, alert intervals, marker channel timing, and the like. The morphology detector 236 is configured to review and analyze one or more features of the morphology of cardiac activity signals, such as the morphology of detected R waves to determine whether to include or exclude one or more beats from further analysis.

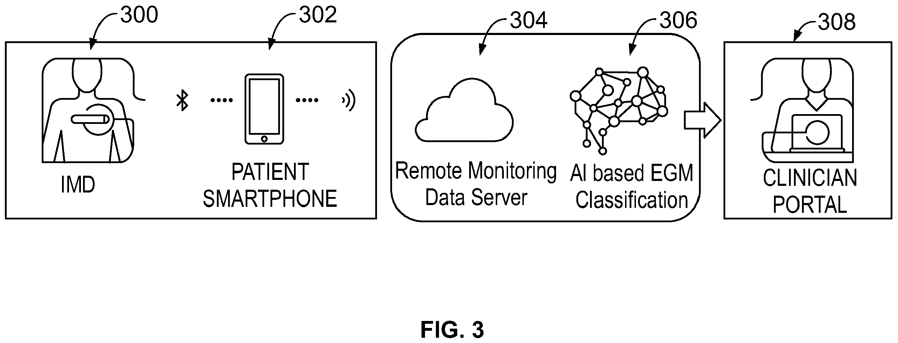

Figure 3 shows a high-level overview of a system formed in accordance with embodiments herein. The system includes an external artificial intelligence (AI) system on a remote monitoring data server. The AI system can include a CNN model as disclosed herein, although other CNN models are contemplated. Implementing this CNN model in a remote monitoring system can significantly reduce EGM episode review burden and streamline the clinic workflow. A further advantage is the ability to provide recommendations for adjusting one or more parameters within the IMD 100, 212 to improve the IMD's detection of true pause events.

At 300, CA signals are analyzed by one or more arrhythmia detection algorithms in the ICM or IMD 100, 212. As discussed herein, the arrhythmia detection algorithms can detect pause. When an arrhythmia is identified, one or more DCA data sets are recorded in connection with the arrhythmia, including device documented markers designating characteristics of interest within the CA signals and/or identifying the nature of the arrhythmia. Once a collection of DCA data sets is stored in the IMD 100, 212, the collection of DCA data sets are wirelessly transmitted from the IMD 100, 212 to a local external device 302 and/or a remote server 304. The data may be transferred when a predetermined volume of DCA data sets has been collected or on a periodic basis. The remote server 304 utilizes one or more CNN models 306 to re-analyze the uploaded collection of DCA data sets. The CNN model 306 identifies valid and invalid subsets of the DCA data sets (also referred to as appropriate and inappropriate subsets). The appropriate or valid subsets include DD markers that correctly characterized the corresponding CA signals, while the inappropriate or invalid subsets include DD markers that incorrectly characterized the corresponding CA signals. Stated another way, the appropriate or valid subset corresponds to correct/positive arrhythmias, such as true pause, while the inappropriate or invalid subset corresponds to incorrect/false arrhythmias, such as false pause. The information concerning the true positives or valid subset is then provided to a clinician portal 308 in various forms, as discussed herein, thus significantly reducing the burden of reviewing all potential EGM pause episodes identified by the IMD 100. In other embodiments, information concerning both the true positive and false positives are provided to the clinician portal 308. The clinician portal 308 may automatically, based on the results, suggest setting adjustments to improve the true positive detection of pause by the IMD 100, 212.

In still further embodiments, the CNN model 306 may be housed within a local external device 302 that additionally or alternatively re-analyzes the uploaded collection of DCA data sets. In this manner, information concerning true positives and false positives can be provided to the patient, alerts can be generated, such as to recommend the patient contact their physician, adjustments, such as within predetermined limits, to the settings of the IMD 100, 212 can be automatically determined and transmitted to the IMD 100, 212 to improve the true positive detection of pause, etc.

Additionally or alternatively, the CNN model 306 may output a confidence indicator (e.g., a probability, likelihood, continuous value between 0 and 1) indicative of a level or degree of confidence that an individual underlying DCA data set represents a true positive or false positive designation of pause. As one example, the numeric indicator may be a continuous value between 0 and 1, where the values close to zero indicate a high confidence that a subcutaneous EGM signal within the DCA data set is not indicative of a pause event, and thus a false positive. The values close to 1 indicate a high confidence that a subcutaneous EGM signal within the DCA data set is indicative of a pause event and thus a true positive. When a DCA data set is not indicative of a pause event, the DCA data set may include CA signals that are indicative of normal sinus rhythm or otherwise. For example, the CA signals within the DCA data set may exhibit an unduly noisy signal that should not be otherwise characterized as an arrhythmia or a normal sinus rhythm. An unduly noisy CA signal within a particular DCA data set, such as those that can result in inappropriate R-wave detection, can be determined using a threshold, such as a desired sensitivity level.

Additionally or alternatively, the CNN model 306 may include a detection threshold that may be changed/tuned by clinicians based on the clinicians needs and various factors. In the foregoing example, where numeric values near 1 indicate a high confidence of true positives and numeric values near 0 indicate a high confidence of false positives, the detection threshold may be lowered (e.g., closer to 0) to increase the sensitivity of the CNN model 306. For example, when the detection threshold is set at .25, the CNN model 306 will identify more false positive DCA data sets, as compared to when the detection threshold is set at .75. For example, it may be desirable to apply a higher level of sensitivity in connection with certain types of pause events that may not occur regularly. In addition, it may be desirable to apply a higher threshold, and thus lower the sensitivity, while increasing the specificity, in connection with other types of arrhythmias that are considered less "critical" to a patient's health and that may occur more often.

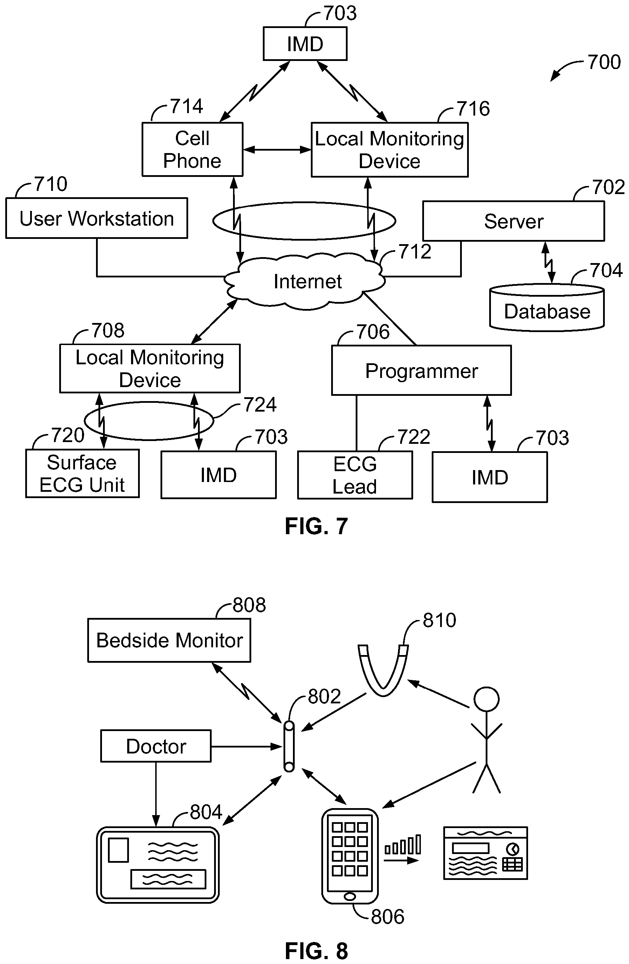

The arrhythmia detection algorithms operating on the IMD 100, 212 and the CNN model(s) operating on the local external device 302 and/or remote server 304 afford two discriminators that work together to form a robust arrhythmia classification system. The system of Figure 3 reduces false positive arrhythmias by implementing a CNN-based confirmation process to provide a second check with respect to IMD 100, 212 declared arrhythmia, and more specifically, IMD 100, 212 declared pause event. The process described herein may be distributed between various devices. For example, one or more of the IMD 100, 212, local external device 302 and/or remote server 304 may include memory to store specific executable instructions and a convolutional neural network (CNN) model that comprises a global average pooling (GAP) layer; and one or more processors configured to execute the specific executable instructions to: obtain device classified arrhythmia (DCA) data sets generated by an implantable medical device (IMD) (e.g., IMD 100, 212) for corresponding candidate arrhythmias episodes declared by the IMD, the DCA data sets including cardiac activity (CA) signals for one or more beats sensed by the IMD; and apply the CNN model to the DCA data sets to identify a valid subset of the DCA data sets that correctly characterize the corresponding CA signals and to identify an invalid subset of the DCA data sets that incorrectly characterize the corresponding CA signals. One or more of the devices illustrated in Figure 3 and Figure 7 discussed further below may further include a display configured to present information concerning at least one of the valid subset or invalid subset of the DCA data sets. Additionally or alternatively, the one or more devices of Figure 3 implement a CNN model that outputs, in connection with each DCA data set, a confidence indicator indicative of a degree of confidence that the corresponding DCA data set represents a true positive or false positive designation of pause.

One or more of the IMDs in Figures 1A and 2A comprise a combination of subcutaneous electrodes configured to collect the CA signals; IMD memory configured to store program instructions; and one or more IMD processors configured to execute the program instructions to: analyze the CA signals and based on the analysis declare candidate pause episodes; generate the DCA data sets including the corresponding CA signals; and a transceiver configured to wirelessly transmit the DCA data sets to an external device. One or more of the external devices in Figures 1A, 2A, 3, and 7 include the memory and the one or more processors and a transceiver, the transceiver configured to wirelessly receive the DCA data sets from the IMD. The server may include memory and the one or more processors, the memory configured to store the collection of the DCA data sets, the one or more processors configured to apply the CNN model to the collection of the DCA data sets.

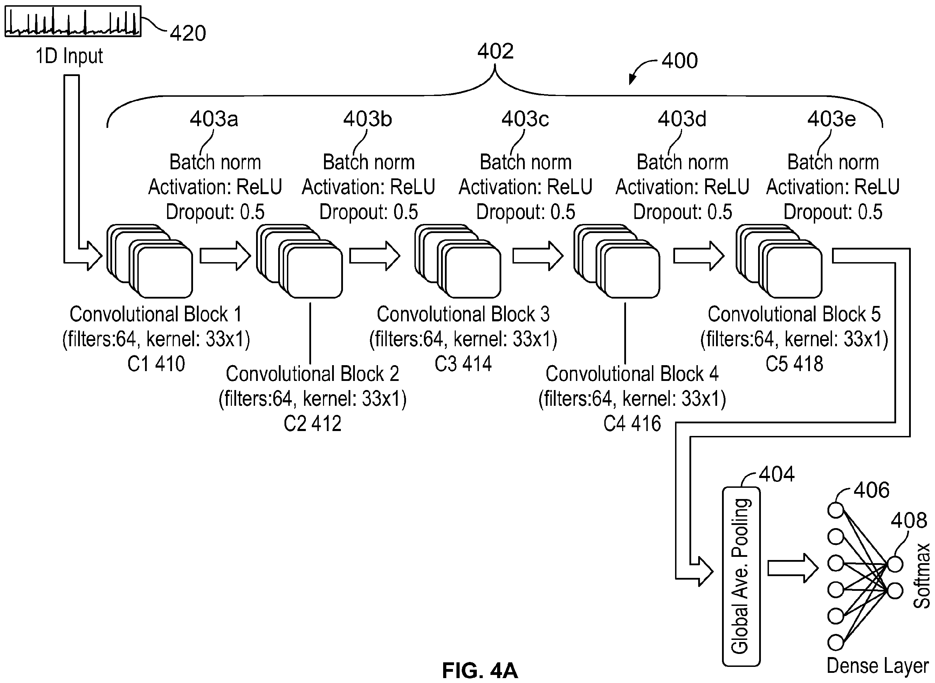

Figure 4A illustrates a summary of an example CNN model utilized in accordance with embodiments herein. Although the CNN model 400 includes a convolutional neural network architecture, it is recognized that the network architecture may differ and/or other types of machine learning models may be utilized.

A fully connected multi-layer neural network is called a Multilayer Perceptron (MLP). MLP is the most frequently used supervised neural network appearing effective in learning complex systems. The MLP architecture is variable, however, it consists of several layers of neurons connected to each other in a feed-forward manner. Each neuron is the weighted sum of its inputs passed through a non-linear function. The number of layers, and the number of neurons is referred to as hyperparameters of a neural network, and these need tuning. Cross-validation techniques are used to find ideal values for these. The weight adjustment training is done via backpropagation. In some cases, deeper neural networks are better at processing data. However, deeper layers can lead to vanishing gradient problems, and special algorithms are used to solve this issue.

CNN is a deep neural network (DNN) architecture usually trained by a gradient-based optimization algorithm. As shown in Figure 4A, the architecture or structure of the CNN model 400 is comprised of five 1-dimensional (1D) convolutional layers 402, global average pooling (GAP) layer 404, a fully connected layer 406, and a Softmax layer 408. It should be understood that other CNN models can include more or less convolutional layers. In some embodiments, each of the convolutional layers 402 have parameters (e.g., Filters:64, Kernal: 33x1) although other sizes are contemplated.

In general, the CNN model 400 includes multiple back-to-back layers connected in a feed-forward manner. The main layers include the convolution layers 402, normalization layers 403, pooling layer (e.g., GAP layer 404), and fully-connected layer 406 (e.g., dense layer). The convolution layers 402, normalization layers 403, and GAP layer 404 are responsible for extracting features, while fully connected layers 406 are in charge of classification. The CNN model 400 excels at automatically learning and extracting relevant features from raw data, eliminating the need for manual feature engineering.

IMD pause EGM episodes in two data sets, e.g., a training data set and a validation data set, are utilized to train and validate the CNN model 400. By way of example only, the training and validation data sets can have a mix of known true pause and false pause events (e.g., the pause EGMs of the training and validation data sets have been expertly adjudicated). In some embodiments, the training data set is ensured to have enough true and false detections to ensure that the data set is not overwhelmed by false positives, while still providing enough examples for the CNN model 400 to learn from. In some cases, the Z-score normalization function is used for each segment.

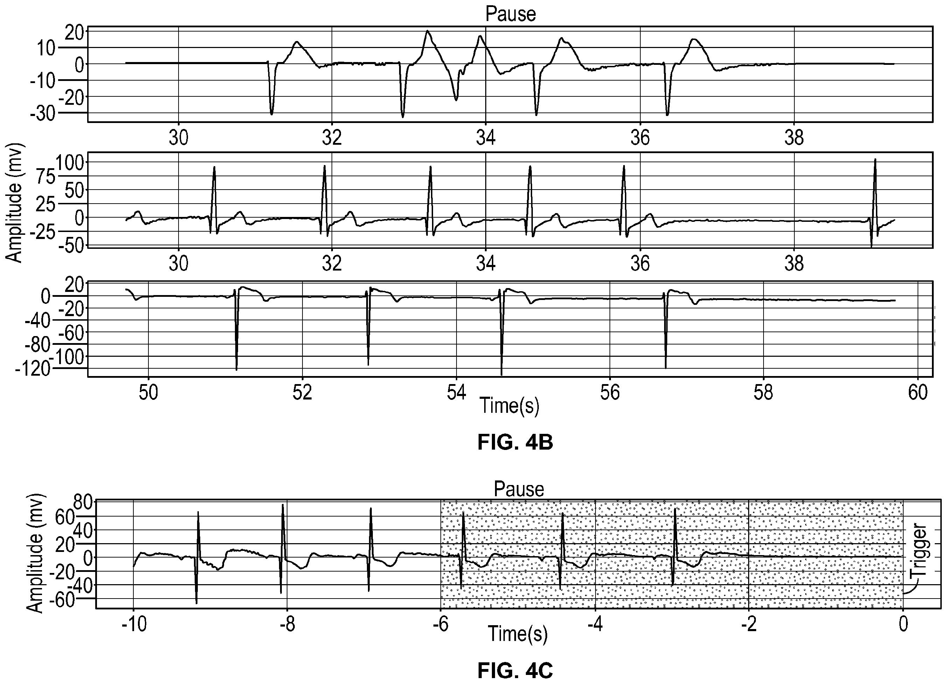

For reference, Figure 4B illustrates three different EGM samples from different IMD devices including pause episodes. The vertical axis plots amplitude in millivolts (mV) and the horizontal axis plots time in seconds (s). To train the CNN model 400, different approximate lengths of SEGM (e.g., 4s, 6s, 8s, 10s, more than 10s, a range between 4s and 10s) before trigger were used. By way of example, Figure 4C shows another SEGM sample including a pause episode in accordance with embodiments herein. The shaded area in Figure 4C indicates 6s before trigger that includes a detected or suspected pause episode. The trigger may be a point in time determined by the IMD 100, 212 wherein the IMD 100, 212 determines a pause episode. a point in time when the patient indicated that they were experiencing a pause episode, etc. The IMD 100, 212 can save the SEGM segment, indicated in Figure 4C as 6s, as a DCA data set.

In some cases, pause adjudication was accomplished using a hybrid approach, such as the approach in "Piorkowski C, Dawoud F, Qu F, Ryu K. Hybrid human and machine learning adjudication approach for evaluating arrhythmia discriminator algorithm performance. APHRS 2019 Poster AP19-00435". In some embodiments, when using the hybrid approach experienced adjudicators confirmed the labels. The hybrid approach leverages machine learning techniques and human verification, is highly accurate, and significantly reduces human adjudication burden. In still further embodiments, some pause episodes were manually adjudicated by experienced adjudicators (e.g., experienced research personnel).

Returning to Figure 4A, a 1D input signal 420 is fed to the first convolutional layer within the five convolutional layers 402 without any feature extraction. In some embodiments the 1D input signal 420 can be a single lead ECG or EGM vector or a 1D set of amplitudes across time. The 1D input signal 420 can be the SEGM of a length as described above (e.g., 4s, 6s, 8s, 10s, more than 10s, a range between 4s and 10s) and during the training phase of the CNN model 400, can be from within a reference device classified arrhythmia (DCA) data set as further discussed below.

Convolutional layers C1 410, C2 412, C3 414, C4 416, and C5 418 each extract different levels of features from the input signal 420. The convolutional layers C2 412, C3 414, C4 416, and C5 418 each receive the output of the previous convolution layer and/or normalization layer, and thus receive a signal based on the input signal 420. The convolutional layers C1 410, C2 412, C3 414, C4 416, and C5 418 perform the convolution operations as expressed in the below equation on the output of a previous layer (or in the case of C1 410 on the input signal 420) using the current convolution kernel [6, 13], and their convolution kernel size is 33x1:

$$x_k^l = f(\sum_{i \epsilon M_k} x_i^{l-1} * \omega_{ik} + b_k)$$

where $M_k$ is the effective range of the convolution kernel, $x_i^l$ represents the output of the $k^{th}$ neuron in layer $l$, $b_k$ is the bias of the $k^{th}$ neuron in layer $l$, $\omega_{ik}$ is the weight kernel between the ith neurons in layer $l$ - 1 and the kth neuron in layer $l$, and $f(\cdot)$ represents the Rectified Linear Unit (ReLU) activation function.

The output of each of the convolutional layers C1 410, C2 412, C3 414, C4 416, and C5 418 is a feature map that can be normalized (e.g., batch norm, batch normalization, batch normalization function), processed by rectified linear unit activation functions (e.g., Activation: ReLU), and/or processed by dropout functions (e.g., Dropout: 0.5) before the subsequent layer receives the data. For example, dropout can be used to randomly remove some of the features to avoid overfitting while activation can use a threshold for determining what features to send to the next level.

By way of example, after applying the five convolutional layers 402 in the proposed architecture as shown in Figure 4A, feature maps are generated that correspond to each respective convolutional layer 410-418. To further process these feature maps, they are typically passed through additional layers, such as batch normalization, activation, and dropout layers. These layers help enhance the performance and robustness of the network by normalizing the feature maps, introducing non-linearity, and preventing overfitting. Batch normalization is a technique that normalizes the activations of the previous layer, ensuring that the inputs to the subsequent layers are standardized and less sensitive to variations in the training data. This normalization helps accelerate the training process and improves the overall stability of the network. Activation functions introduce non-linearity to the feature maps, enabling the network to learn complex patterns and relationships in the data. Common activation functions include ReLU (Rectified Linear Unit), sigmoid, and tanh. Dropout is a regularization technique used during training to prevent overfitting. It randomly sets a fraction of the activations in the feature maps to zero during each training iteration. This forces the network to learn more robust and generalized representations by preventing the reliance on specific activations. By passing the feature maps through batch normalization, activation, and dropout layers, the network can further refine the learned representations and increase its ability to extract meaningful features from the input data.

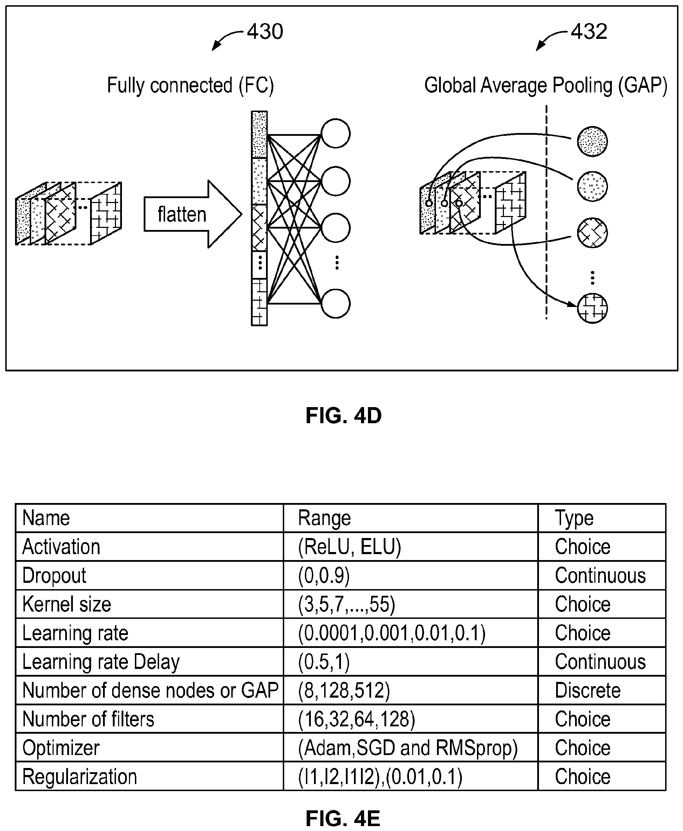

Global average pooling (GAP) layer 404 is a pooling operation designed to replace fully connected layers in classical CNNs. The GAP layer 404 receives output from all of the convolutional layers 402 substantially simultaneously as shown in Figure 4D. The GAP layer 404 in the CNN model 400 computes the average value of each feature map, reducing the spatial dimensions of the data to a fixed-length vector. This technique offers several advantages, including dimensionality reduction, translation invariance, preservation of spatial hierarchy, and increased interpretability. By reducing the dimensionality, the model becomes more efficient and less prone to overfitting. Translation invariance ensures the model's robustness to spatial shifts. The spatial hierarchy preservation captures important spatial patterns, while the resulting fixed-length vector enhances interpretability by highlighting feature presence or absence. Overall, the GAP layer 404 improves computational efficiency, generalization, and interpretability in the CNN model 400.

In some embodiments, one feature map is generated for each corresponding category of the classification task in the last convolutional layer. Instead of adding fully connected layers on top of the feature maps, the average is taken of each feature map, and the resulting vector is fed directly into the Softmax layer 408.

The output of the Softmax layer 408 is a continuous value between 0 and 1, where values close to zero indicate high confidence that an SEGM does not correspond to an arrhythmia (e.g., pause) and is a false positive, and values close to 1 indicate high confidence of a true positive. This enables the system to be tuned according to clinical needs. For example, the detection threshold can be lowered so that the system is very sensitive to certain types of critical arrhythmias that might not occur regularly. For less critical arrhythmias that might occur more often, the classification threshold (e.g., detection threshold) can be increased for higher specificity.

Figure 4D shows a comparison of a fully connected layer 430 and a GAP layer 432 in accordance with embodiments herein. One advantage of the GAP layer 432 over the fully connected layer 430 is that the GAP layer 432 is more native to the convolution structure by enforcing correspondences between feature maps and categories. Thus, the feature maps can be easily interpreted as categories confidence maps. Another advantage is that there is no parameter to optimize in the GAP layer 432 thus overfitting is avoided at this layer. Furthermore, the GAP layer 432 sums out the spatial information, thus it is more robust to spatial translations of the input. In some embodiments, less epochs were needed for the GAP layer 432 to have the best results without overfitting.

By using the GAP layer 432, each of the activation maps in the final layer preceding the GAP layer 432 acts as a detector for a different pattern in the input, localized in space. To get the class activation map corresponding to an image, the detected patterns are transformed to detected objects. This transformation is done by noticing each node in the GAP layer 432 corresponds to a different activation map, and that the weights connecting the GAP layer 432 to the final dense layer encode each activation map's contribution to the predicted object class. To obtain the class activation map (e.g., classification), the contributions of each of the detected patterns in the activation maps are summed, where detected patterns that are more important to the predicted object class are given more weight.

In some embodiments, referring to the fully connected layer 430, after the last convolutional layer, fully connected layers are added on top of the feature maps. Each neuron in the fully connected layer 430 is connected to every neuron in the previous layer, effectively flattening the spatial structure of the feature maps (e.g., flattened vector). This allows the network to learn complex relationships between features across the entire image. The output of the fully connected layer is then fed into a Softmax layer 408 for classification. Referring to the GAP layer 432, instead of using fully connected layers, the average is taken of each feature map individually. This involves computing the average value of each feature map across its spatial dimensions (e.g., width and height), resulting in a single value per feature map. These averaged values are then used as a compressed representation of the feature maps. The resulting vector, which contains the average values from each feature map, is directly fed into the Softmax layer 408 for classification. The use of Global Average Pooling has several advantages. It reduces the number of parameters in the network since there is no need for fully connected layers, which can help alleviate overfitting. It also retains the spatial information present in the feature maps to some extent, as the averaging operation is performed individually on each feature map. This can be beneficial for tasks where spatial relationships are important, such as EGM signal classification.

For the last layer, the Softmax layer 408 was used as the activation function in the output layer of the CNN model 400 to predict a binomial probability distribution. The Softmax formula is as follow:

$$\sigma(\vec{Z})_i = \frac{e^{Z_i}}{\sum_{j=1}^{k} e^{Z_j}}$$

where all the $Z_i$ values are the elements of the input vector and can take any real value. $k$ is the number of classes. The term on the bottom of the formula is the normalization term which ensures that all the output values of the function will sum to 1, thus constituting a valid probability distribution.

Deep neural networks lead to vanishing gradient problems. Vanishing gradient refers to the fact that in deep neural networks, the backpropagated error signal (gradient) typically decreases exponentially as a function of the distance from the last layer. In other words, the useful gradient information from the end of the network fails to reach the beginning of the network. Batch normalization is a normalization technique that is applied to the input (current) batch of data, which is the output of the previous convolutional layer. Omitting the rigorous mathematical details, batch normalization can be simply visualized as an additional layer in the network that normalizes the data (using a mean and standard deviation) before feeding it into the hidden unit activation function. It can solve the problem of vanishing gradients when using with ReLU activation function.

To do that, the average value is taken out of each layer called $\mu_B$. This is calculated as the sum of all values of layer $x_i$ divided by average of all m values.

$$\mu_B = \frac{1}{m}\sum_{i=1}^{m} x_i$$

The variance $\sigma_B^2$ can then be calculated as follows: 1. Subtracting the $\mu_B$ from every value which is the deviation of every value and take the square for squared deviation; and 2. Sum up the results of doing that for each of the values, then divide by the number of values m, to get the average, or mean squared deviation:

$$\sigma_B^2 = \frac{1}{m}\sum_{i=1}^{m}(x_i - \mu_B)^2$$

The standard deviation can be found as the sum of mean squared deviation and epsilon under root. The epsilon is a constant value as small as 0.001. This is added to avoid cases of division by zero and also to increase variance. The variance of a population is typically more than the variance for any sample taken from that population, especially in cases of small sample size where values are near the peak of the population distribution, so increasing the variance a little bit for each batch helps take that into account. Now that the mean and standard deviation are calculated, normalize as follows:

$$\hat{x}_i = \frac{x_i - \mu_B}{\sqrt{\sigma_B^2 + \varepsilon}}$$

The normalized values are then multiplied by $\gamma$ and added with $\beta$. These are the learnable parameters which serve in further scaling the normalized values. The final batch normalized value is as follows:

$$y_i = \gamma\hat{x}_i + \beta$$

The batch normalization can be applied before and after the activation function. However, in some embodiments better results are achieved when the batch normalization is applied before the activation function. During training, the mean and standard deviation are calculated using samples in the mini batch. However, in testing, it does not make sense to calculate new values. Hence, batch normalization uses a running mean and running variance that is calculated during training. Batch normalization can result in a faster training time, and the ability to use higher learning rates without vanishing or exploding gradients is promising as well.

The term "dropout" refers to dropping out the nodes (input and hidden layer) in a NN. All the forward and backward connections with a dropped node are temporarily removed, thus creating a new network architecture out of the parent network. The nodes are dropped by a dropout probability of p. For instance, if the hidden layers have 1000 neurons (nodes) and a dropout is applied with drop probability = 0.5, then 500 neurons would be randomly dropped in every iteration (batch). Generally, for the input layers, the keep probability, i.e., 1- drop probability, is closer to 1. In some embodiments, the keep probability is approximately 0.8. For the hidden layers, the greater the drop probability the sparser the model, where approximately 0.5 may be the most optimized keep probability, that states dropping 50% of the nodes. In the overfitting problem, the CNN model 400 learns the statistical noise. To be precise, the main motive of training is to decrease the loss function, given all the neurons. Therefore, in overfitting, a unit may change in a way that fixes up the mistakes of the other units. This leads to complex co-adaptations, which in turn leads to the overfitting problem because this complex co-adaptation fails to generalize on the unseen data set. If dropout is used, it prevents these units from fixing up the mistake of other units, thus preventing co-adaptation, as in every iteration the presence of a unit is highly unreliable. So, by randomly dropping a few units (nodes), it forces the layers to take more or less responsibility for the input by taking a probabilistic approach. During the inference, a dropout layer is not used. This means that all the units are considered during the prediction step. But, because of taking all the units/neurons from a layer, the final weights will be larger than expected and to deal with this problem, weights are first scaled by the chosen dropout rate. With this, the network would be able to make accurate predictions. In some cases, if a unit is retained with probability p during training, the outgoing weights of that unit are multiplied by p during the prediction stage.

Hyperparameter optimization and training

When implementing NN, there are several choices (or hyperparameters) that must be set prior to training- those range from the type of architecture to the depth and width of the layers, through to the neuronal activation-function in the different layers, and so on. As choosing hyperparameters arbitrarily is likely to lead to suboptimal results, a three-way split of the data into training, validation, and test sets is created to identify reasonable architectures and parameter ranges. Then, guided by those pre-established ranges, NN optimization can be conducted via a KerasTuner and grid search. By employing this procedure, a large space of possible models can be evaluated and many configurations tested.

Figure 4E illustrates a table of exemplary hyperparameters of interest and the range of available options during the search in accordance with embodiments herein. The hyperparameters of interest consisted of the activation function, dropout percentage, learning rate, learning rate decay, nodes per layer, and the optimizer. Additional hyperparameters for convolutional models included the number of filters, the kernel size, and regularization. It should be understood that other hyperparameters can be used.

In some embodiments, for the dataset the following procedure may be used. For each set of hyperparameters sampled in the search, the data can be partitioned into a training (80%) and validation set (20%). The proposed architecture was thus trained on the training dataset. Then, to evaluate the architecture, the validation accuracy scores can be checked for different thresholds. The network architecture with the highest average accuracy score for all the thresholds can be selected. In some cases, all or some networks can be trained for 1000 epochs using an early stopping condition. In some cases, when the accuracy on the validation set did not improve for 25 epochs, training stopped. All models can be trained using ten-fold cross-validation. This cross-validation procedure requires a given model to be trained ten distinct times (e.g., re-initializing the network parameters each time) and ensures that i) different subsets of the data are used for training and testing, and ii) each data point serves as part of the training set (e.g., nine times) and in the test set (e.g., once). In some cases, when cross validation was performed, the data partitions used were not used during the hyperparameter search.

Data augmentation and regularization used during training

In order to avoid overfitting while training the neural network, the SEGMs in the training set are augmented. By augmenting the data, the CNN model 400 is exposed to a wider range of examples, helping it learn more robust and invariant representations. Data augmentation is particularly useful when training deep learning models with limited labeled data, as it effectively increases the effective size of the training set without incurring additional data collection or labeling costs.

Generally, in machine learning, but especially for deep learning, the classification accuracy tends to depend on the amount of training data; limited training data typically leads to low accuracy. Data augmentation comprises the systematic generation of new samples to augment an existing data set by transforming existing samples in a manner that increases the accuracy and stability of classification. Exposing the classifiers to varied representations of its training samples typically makes the model more invariant and robust to such transformations when attempting to generalize the model to new data sets. In some embodiments, popular techniques for data augmentation of signals falls into five categories: noise addition, generative adversarial net (GAN), sliding window, Fourier transform, and others such as reversing, scaling, zoom in and zoom out. In some embodiments, different data augmentation methods are selected based on the data set.

For example, data augmentation can be used to avoid overfitting and increase the performance of the CNN models. For augmenting the training data set using reversing, the input signal can be flipped by multiplying by -1, effectively creating a mirrored version of the original data. This augmentation technique provides additional variations in the temporal characteristics of the EGM signals (e.g., magnification factor=2, percentage 100%). The intuition behind this data augmentation is that the IMD 100, 212 can be implanted upside down and may move within the body, resulting in negative R-peaks in the data set. In some cases, reversing the original training data set effectively doubles the training data set. In some embodiments, the magnification factor for data augmentation can be approximately 2.

By way of example only, referring to noise addition, in some embodiments a Gaussian noise layer (mean = 0, std =0.05) can be added to mitigate overfitting, and as it is a regularization layer, it is only active at training time. In other embodiments, other various types of noise such as Poisson, Salt and Pepper noise, etc., can be added with different parameters (for instance: mean ($\mu$) and standard deviation ($\sigma$)) to the raw EGM signal. In still further embodiments, Gaussian noise with different parameters (mean = 0, standard deviation $\sigma$ = (0.01, 0.1, 0.2, 0.5) can be added to all channels of raw EGM signal.

In some embodiments, data augmentation can include inserting one or more p waves during a pause interval at a previous R-R interval. Referring to Figure 4C for reference, the augmentation would include adding one or more p waves of the same or different morphologies into the pause duration or pause interval, starting from the last p wave before the QRS wave at around -3 seconds and adding one or two p waves prior to the trigger using an RR interval that can be selected (e.g., average of the previous few, between 2 to 10 cycles) to get an approximate p wave morphology. This can be used to augment true pause episodes where the p wave is not present during the pause duration.

In other embodiments, data augmentation can include stretching or shrinking a duration of the pause interval. Referring again to Figure 4C for reference, the signal can be stretched starting approximately 500 ms after the last QRS, around -2 seconds, and extending/shrinking the pause duration or pause interval to change the duration of the pause interval to another physiologically valid duration, e.g., between 3 to 10 seconds. This can be accomplished by expanding or compressing and then interpolating using a spline, linear interpolation, in some cases by removing segments, or by replicating short segments on the time axis and interweaving the replicated segments.

In some embodiments, as the dataset expands with more samples, it is beneficial to continue employing one or more data augmentation methods. By incorporating various augmentation techniques, the robustness of the trained model can be further increased and its ability to generalize to unseen data improved. In addition to signal reversal, a Gaussian noise layer can be utilized during the training phase. A Gaussian noise layer refers to a layer that adds random Gaussian (normally distributed) noise to the input data during the training process. This noise layer helps introduce a stochastic element into the neural network, which can be also beneficial for regularization and generalization. This noise layer introduces random variations to the input data, mimicking the presence of background noise commonly encountered in EGM recordings. By including the Gaussian noise layer, the processor(s) effectively performs a form of random data augmentation. This augmentation strategy aids in training the model to be more resilient to noise and enhances its ability to accurately classify EGM signals in real-world scenarios. Further, by combining signal reversal and the Gaussian noise layer, the training dataset is enriched with augmented samples that capture a broader range of variations present in EGM signals. This augmented dataset facilitates better model training, improves generalization, and enhances the model's capacity to handle noisy or distorted EGM data during inference.

Hierarchical feature extraction

The CNN model 400 utilizes hierarchical layers of filters that automatically capture different levels of features, such as low-level features, high-level features, as well as feature levels on a continuum from low-level to high-level, from the input data. In the case of cardiac rhythm analysis, these filters can effectively capture both subtle and complex patterns associated with different types of episodes. By automatically learning and extracting relevant features, the CNN model 400 enhances the accuracy and robustness of the classification process.

The interpretation of the level of a feature can be defined within the system and can vary based on the specific problem and dataset. In some embodiments, low-level features in EGM signals can refer to basic waveform patterns, such as P-waves, QRS complexes, T-waves, etc. In some cases, these features may be relatively easy to identify visually since they exhibit distinctive shapes and amplitudes. However, it is important to note that the visual perception of these features might vary based on the individual's expertise and familiarity with EGM interpretation, and thus different people may report different results based on visual perception.

The order of feature extraction can depend on the problem and the network architecture being used. In some CNNs, the feature extraction process is hierarchical, where lower layers learn low-level features, and higher layers learn more complex and abstract features. It is common to start with low-level features and progressively move towards higher-level features in a bottom-up fashion. This order helps capture local patterns before capturing more global or context-dependent patterns. In this example, the convolutional layer C1 410 extracts lower level features compared to the convolutional layers C2-C5 412-418, which each extract progressively higher level features than the previous convolutional layer. In other embodiments, the convolutional layer C1 410 extracts higher level features than the convolutional layer C2 412, which extracts higher level features than the convolutional layer C3 414, and so on. In still further embodiments, each of the convolutional layers C1-C5 extracts a different level of features.

Increased data utilization

The CNN model 400 can leverage large amounts of data to improve performance. With more data, the model 400 can learn more diverse patterns and generalize better to new instances. This is particularly advantageous in the medical field, where access to substantial labeled data sets can be challenging. By utilizing more features on CNNs, such as patient specific data, the model can capture personalized characteristics, leading to improved accuracy in classifying cardiac episodes.

Performance measurement

Common quantitative metrics can be used for evaluation of classifiers' performance. The classification resulted from a learning method can be either abnormal case or normal, named as positive class or negative class, respectively. The result of the prediction can also be either true or false, implying correct prediction or incorrect prediction, respectively. For example, classification can be summarized into four possible states: a) True positive (TP): Correct prediction of positive class, b) True negative (TN): Correct prediction of negative class, c) False positive (FP): Incorrect prediction of positive class, or d) False negative (FN): Incorrect prediction of negative class.

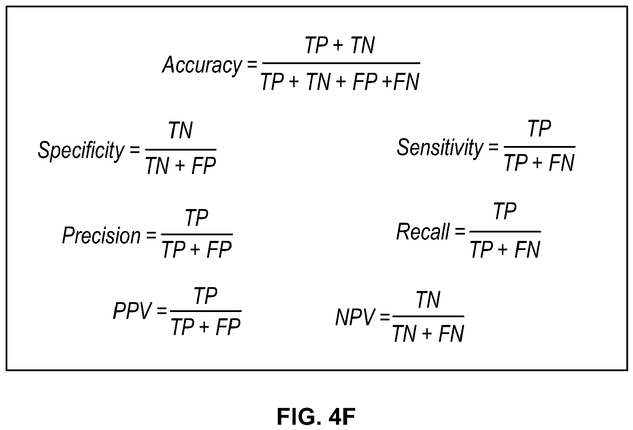

Based on the classification's predictions (e.g., TP, TN, FP, FN), the Accuracy, Specificity, Sensitivity, Precision, Recall, Positive Predictive Value (PPV), Negative Predictive Value (NPV) and Area under the Curve (AUC) can be calculated. Figure 4F shows example equations that may be used to calculate a plurality of metrics in accordance with embodiments herein. Additionally or alternatively, the performance of a classification model at all classification thresholds can be evaluated by receiver operating characteristic curve (ROC). In some embodiments, sensitivity can be used to select the best CNN model with best hyperparameters. In other embodiments, to select the best CNN model, high sensitivity, specificity and F1-score (or other measure of the model's accuracy) and the high AUC of ROC curve can be used.

Personalization

CNNs have the capability to learn and adapt to individual patient data. By training the model on a diverse data set that includes patients with varying cardiac rhythms, the CNN model can capture patterns and nuances that are specific to each patient. This personalized approach enhances the accuracy of the classification algorithms by accounting for patient-specific variations and reducing false positives.

Further, training data associated with certain conditions, unique conditions, pathologies, and/or medications may be used to train or tune the CNN model 400. In some cases, certain episodes, such as of specific pause episode(s), may be used to tune the CNN model 400 or create a tuned CNN model tailored to the particular episode(s). In some embodiments, the CNN model 400 can be tuned based on one or more patients' data. In other embodiments, a tuned CNN model, such as a copy of the main class CNN model 400 that has been further tuned to include parameters that are better tailored to one or more patients, can be created and used to analyze data sent by those one or more patients to the data server 304.

In some embodiments, one or more hyperparameters of the CNN model 400, such as filter size, kernel size, etc., may be adjusted to generate a tuned CNN model for one or more patients.

In a personalized CNN model, the EGM data passes through the CNN model to capture relevant features. However, in addition to the EGM data, extra information such as demographic features and age can be incorporated into the personalized CNN model. These additional features can be combined with the learned EGM features at the fully connected layer 406. By incorporating this supplementary information, the personalized CNN model 400 can leverage the demographic characteristics and age to enhance its performance and tailor the predictions to the individual patient.

In some embodiments, N personalized CNN models can be created based on different patient categories, or combination of categories, as described below. Physicians can evaluate and determine the most suitable model for specific patients based on their individual features and/or combination of features: i) Age-related features: Besides age itself, additional age-related features like age group (e.g., young, middle-aged, elderly), age range, or indicators for different life stages can be added; ii) Gender: Including gender as a feature can help capture any gender-specific variations in EGM abnormalities; iii) Body Mass Index (BMI): BMI is a measure of body fat based on height and weight which can provide insights into the potential impact of body composition on EGM abnormalities; iv) Ethnicity: Ethnicity can play a role in certain cardiovascular conditions, and including this feature may help capture any population-specific variations; v) Medical history: Incorporating relevant medical history features such as hypertension, diabetes, previous heart conditions, or other relevant chronic diseases can provide valuable context for identifying EGM abnormalities; vi) Medication usage: Information about the medication that a person is currently taking can be included, especially if certain medications are known to affect EGM readings or can be indicative of underlying conditions; vii) Lifestyle factors: Including features related to lifestyle choices such as smoking status, alcohol consumption, exercise habits, or occupation can provide additional insights into potential risk factors; and viii) Family history: Incorporating information about family history of cardiovascular diseases or EGM abnormalities may help identify individuals with a higher predisposition to such conditions.

In other embodiments, physicians can collaborate, such as with data scientists, to iteratively refine the personalized CNN model (e.g., iterative refinement). Physicians can provide feedback on false positives, false negatives, and areas where the CNN model may benefit from further fine-tuning to align with real-world clinical scenarios. By involving physicians throughout the programming process, the personalized CNN model can benefit from their clinical expertise and domain knowledge. This collaboration helps ensure that the personalized CNN model aligns with medical standards, enhances accuracy, and is clinically relevant, which can improve patient care and outcomes beyond the improvements already experienced by use of the CNN model 400.

In still further embodiments, physicians can create a personalized CNN model based on a more general CNN model and/or feed personalized information into a personalized CNN model. For example, the physician may select an option offered on, for example but not limited to, a graphical user interface (GUI), to personalize a CNN model, select the CNN model they wish to personalize, and select one or more options (e.g., categories, features) offered on the GUI. In other cases, data may be input using voice, gesture, a camera, a microphone, etc. In some cases, the physician may tailor the personalized CNN model as a patient changes medications and/or dosages of medications, is diagnosed with a new condition, etc. The physician may then be able to further tailor separate ones of the personalized CNN models to one or more of a specific patient's needs, further personalizing medical care.

Transfer learning

CNNs can benefit from transfer learning, where knowledge gained from training on one data set can be transferred to a related task or data set, without updating the feature extraction of the five convolutional layers 402. In the context of IMDs 100, 212, transfer learning enables the development of CNN models for automatic classification of different cardiac episodes. For instance, a CNN model trained on a large data set for pause detection can be fine-tuned and adapted to classify brady episodes in IMDs 100, 212. By leveraging the pre-trained knowledge and general features learned from related tasks, transfer learning reduces the need for extensive labeled data specific to each episode type and accelerates the development of accurate algorithms.

In some embodiments, the hyperparameters of the five convolutional layers 402 of the CNN model 400 can be frozen or fixed and the fully connected layer 406 can be tuned. Transfer learning can utilize streaming data and new data from the patient that has not been adjudicated by expert adjudicators.

In some cases, transfer learning can be used to update the CNN model 400 on a regular basis, such as weekly, monthly, etc., when additional data is received. Updating the CNN model 400 in this manner will improve the performance of the model over time.

During transfer learning, the new data travels through the parameters of a pre-trained CNN model in a process known as fine-tuning. The steps below generally summarize the process of fine-tuning, although it should be understood that other steps may be used that are not included, and some steps may be omitted:

i) Pre-trained Model: Transfer learning begins with a pre-trained CNN model (e.g., CNN model 400) that has been trained on a large data set for a specific task, such as pause detection, image classification, natural language processing, etc. This pre-trained CNN model contains learned parameters (e.g., weights, biases) that capture general patterns and features from the original data set it was trained on;

ii) Frozen Layers: In transfer learning, the initial layers of the pre-trained CNN model, which capture low-level and general features, are usually frozen. This means that their parameters are not updated during the training process, and their learned representations are preserved;

iii) New Data: The new data, which typically comes from a different but related task or domain, is introduced to the transfer learning pipeline. This data could be a smaller data set specific to the new task (e.g., brady episodes) or additional data from the original task (e.g., additional pause episodes);

iv) Forward Pass: During the forward pass, the new data is passed through the pre-trained CNN model, starting from the input layer. As the data propagates through the network, it undergoes transformations based on the learned parameters. The frozen layers retain their original parameters and do not update based on the new data, whereas the unfrozen layers (usually the top layers) are trainable and will be updated;

v) Backpropagation: After the forward pass, the loss or error between the predicted output and the ground truth labels is computed. Then, backpropagation is performed to calculate the gradients of the loss with respect to the trainable parameters of the unfrozen layers;

vi) Gradient Descent: The gradients obtained from backpropagation are used to update the parameters of the unfrozen layers through an optimization algorithm, typically gradient descent. The optimizer adjusts the weights and biases of these layers to minimize the loss, making the model more suitable for the new task;

vii) Training Iterations: The process of forward pass, backpropagation, and parameter update is repeated for multiple iterations or epochs, allowing the CNN model to gradually adapt to the new data. This process refines the learned representations in the unfrozen layers to align with the specific patterns and features present in the new task. By allowing the new data to propagate through the unfrozen layers while keeping the frozen layers intact, transfer learning leverages the previously learned knowledge to accelerate training and improve performance on the new task.

During transfer learning, the process of updating the parameters of the CNN model 400 is typically done automatically through an optimization algorithm, such as gradient descent. The CNN model 400 does not manually change the parameters; rather, it learns to update them based on the new data and the defined optimization objective. The optimization algorithm, guided by the loss function, computes the gradients of the loss with respect to the model's parameters. These gradients represent the direction and magnitude of parameter updates required to minimize the loss.

The optimizer then adjusts the parameters using these gradients. In the context of transfer learning, the pre-trained model's parameters are usually partially frozen, and only the parameters in the unfrozen layers are updated. The frozen layers retain their learned representations and do not change during training on the new task or data set.

By allowing the optimization algorithm to automatically update the parameters, the model adapts its representations and fine-tunes them to better suit the new task or data set. This automatic adjustment process based on the new data helps the CNN model capture task-specific patterns and improve its performance. In some embodiments, the below code may be used:

```
# Freeze the layers up to a certain layer
freeze_layers_until = 5
for layer in pretrained_model.layers[:freeze_layers until]:
layer.trainable = False
```

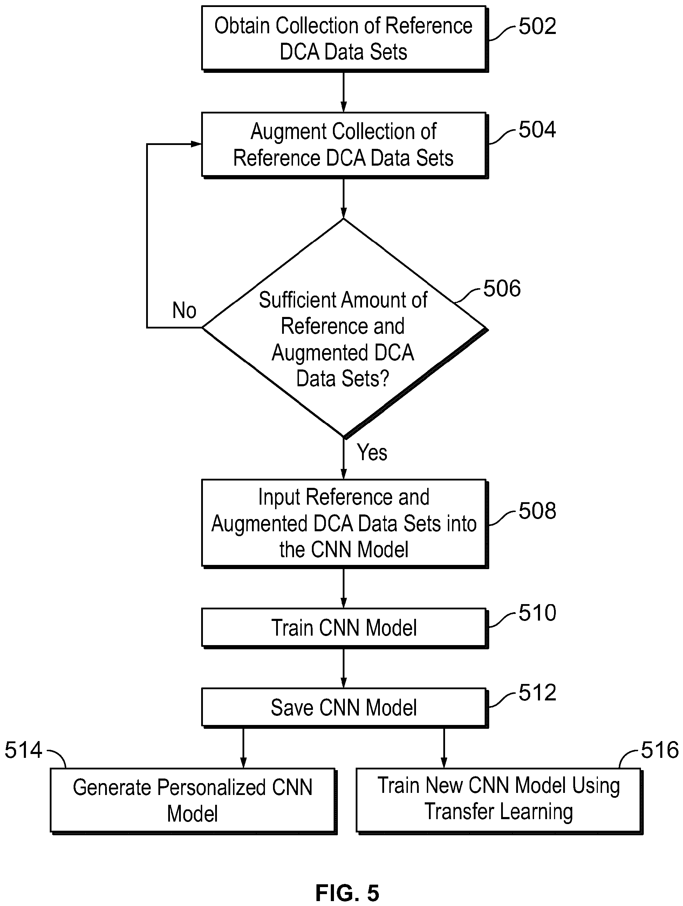

Figure 5 illustrates a process for training/building a convolutional neural network (CNN) model to analyze DCA data sets, relative to an arrhythmia of interest such as pause, in accordance with embodiments herein. The operations of Figure 5 may be implemented by hardware, firmware, circuitry and/or one or more processors housed partially and/or entirely within an IMD, a local external device, remote server, a combination thereof, and/or more generally within a healthcare system. Optionally, the operations of Figure 5 may be partially implemented by an IMD and partially implemented by a local external device, remote server or more generally within a healthcare system. For example, the IMD includes IMD memory and one or more IMD processors, while each of the external devices/systems (ED) (e.g., local, remote or anywhere within the healthcare system) include ED memory and one or more ED processors.

At 502, one or more processors of the system obtain a collection of reference DCA data sets. In some embodiments, each of the reference DCA data sets includes far field reference CA signals (e.g., SEGM signals) for one or more beats sensed along a sensing vector between a combination of subcutaneous electrodes that are not located transvenously. The subcutaneous electrodes may be provided on or coupled to an IMD. For example, the subcutaneous electrodes may be provided on the housing of an IMD 100 and/or provided on a non-transvenous lead coupled to a subcutaneous IMD. Each of the DCA data sets further includes one or more device documented (DD) markers, generated by the IMD, characterizing the CA signals within the corresponding DCA data set. A reference DCA data set includes CA signals for one or more reference cardiac beats known to include a corresponding arrhythmia of interest, such as pause. The reference DCA data set further includes CA signals for reference cardiac beats known to be normal and to not include the arrhythmia of interest.

Additionally or alternatively, an ICM, such as a leadless cardiac monitor in U.S. 9,949,660, senses and classifies cardiac activity. Additionally or alternatively, a leadless IMD, such as in U.S. patent 9,216,285, senses cardiac activity. Additionally or alternatively, a transvenous IMD, such as in U.S. Patent 8,391,980, senses cardiac activity. For implantable devices that do not classify cardiac activity within the device, the cardiac activity can be transmitted to an external device for classification and/or further processing to include one or more DD markers, generate a reference DCA data set including CA signals for one or more reference cardiac beats known to include pause and/or a reference DCA data set including CA signals for reference cardiac beats known to not include pause.

In some embodiments, a 6 second EGM strip (other time lengths are contemplated) may be utilized as one reference DCA data set where the 6 second EGM strip is known to include a pause episode. Multiple separate 6 second EGM strips are collected at different points in time for one patient, for a patient population, recorded by a variety of device types, device placements, device orientations and the like, where each of the separate 6 second EGM strips have CA signals that are known to include corresponding arrhythmias of interest (e.g., pause). As a further example, a second collection of 6 second EGM strips are obtained where the second collection includes reference DCA data sets that are known to correspond to normal rhythms. The collection of reference DCA data sets may be recorded for one patient, a patient population, recorded by a variety of device types, device placements, device orientations and the like.

The reference DCA data sets include device documented markers (e.g., pause designators, R-wave markers, P-wave markers, RR intervals, AF designators) that identify the cardiac beats sensed by the device within the series of cardiac events. The cardiac activity data may have been previously acquired and stored in memory of an implantable or external monitoring device, implantable or external therapy delivery device, programmer, workstation, healthcare network or other system. When the reference DCA data sets have been previously acquired, the obtaining operation at 502 represents accessing and reading the previously stored reference DCA data sets from one or more memory locations.

The CA signals are for one or more cardiac events spanning over various periods of time. As one example, multiple segments or sets of the cardiac activity data may be collected, where each segment/set is for an interval that is 6 seconds to 5 minutes in length. Optionally, the segments may include one or more IMD declared pause episodes. As another example, each of the segments or sets of the cardiac activity data may be collected for an interval that begins 10-60 seconds before an episode of interest (e.g., a pause episode) and that ends 10-60 seconds after the episode of interest. The CA signals may include one or multiple arrhythmia episodes. The DCA data sets obtained at 502 may include one or more detected arrhythmia episodes and/or one or more cardiac beats confirmed to be normal with no arrhythmia episodes.

The DCA data set obtained at 502 may correspond to one continuous series of cardiac events (e.g., 1 continuous series for 6 seconds to 5 minutes) and/or separate sets of cardiac events (5, 10 or more separate series, each for 6 seconds to 5 minutes of cardiac events).

Collection and analysis of CA signals by the IMD may be initiated automatically when the IMD detects an arrhythmia episode of interest (e.g., pause). Additionally or alternatively, the IMD may collect and analyze CA signals in response to a user-initiated instruction or clinician instruction. For example, a user or clinician may utilize a smart phone, programmer or other portable device to establish a communications session with the IMD and instruct the IMD to begin to collect and analyze cardiac signals, such as when the patient is experiencing discomfort, feeling faint, a rapid heart rate, during a clinic visit, etc.

At 504, the one or more processors augment the collection of reference DCA data sets, by generating synthetic DCA data sets based on the reference DCA data sets, to enlarge the total number of DCA data sets. Each of the synthetic DCA data sets is based on, but is different in some manner from, a corresponding reference DCA data set. Herein, the combination of the reference and synthetic DCA data sets are referred to as the "augmented collection of DCA data sets" and is utilized when training the CNN model 400. By augmenting the collection of DCA data sets, embodiments herein avoid overfitting while training the CNN model 400. The augmentation process may be performed in advance of use, or in real-time or substantially in real-time while the augmented collection of DCA data sets is supplied to the CNN model 400.

The CNN model 400 may be trained based on raw CA signals from the augmented collection of reference and synthetic DCA data sets. As discussed above, the augmentation may be implemented in various manners. As nonlimiting examples, augmentation such as noise addition and/or reversing may be used to generate synthetic DCA data sets based on the reference DCA data sets. Other augmentation includes magnification, inserting one or more p waves during a pause interval at a previous R-R interval, and/or stretching or shrinking a duration of a pause interval,

Additionally or alternatively, other types of augmentation may be used, and the order in which types of augmentation are applied may be varied to generate additional synthetic DCA data sets. For example, data sets that have been augmented by noise may be further augmented by reversal, and vice versa. In some embodiments, using more than one or a plurality of different types of augmentation can facilitate the creation of a larger amount/number of synthetic or augmented DCA data sets.

At 506, the one or more processors determine whether a sufficient amount of augmentation has been applied, namely whether a sufficient amount of synthetic DCA data sets have been generated. If not, flow returns to 504 where additional synthetic DCA data sets are generated. Once a desired amount of synthetic and reference DCA data sets are available, flow moves to 508. In some embodiments, if additional reference DCA data sets are needed, flow returns to 502. The decision at 506 enables the process to build numerous types of synthetic DCA data sets based on the reference DCA data set in order to expand the overall collection of DCA data sets by a predetermined amount. For example, it may be desirable to double, triple or otherwise enlarge the original collection of reference DCA data sets by a factor of X.

As another example, different combinations may be defined to achieve different percentages of each type of augmentation within the overall data set. For example, it may be desirable that 50% of the data is overall reference DCA data sets, 20 - 30% are synthetic DCA data sets generated from noise addition of the original DCA data sets, 10 -15% are synthetic DCA data sets generated by reversal of the original DCA data sets, and the remainder is generated using both noise addition and reversal, including any and all permutations and combinations thereof. In other embodiments, a percentage of the synthetic DCA data sets are generated by inserting one or more p waves during the pause interval and/or stretching and/or shrinking a duration of the pause interval. It should be noted that different percentages of reference DCA data sets, synthetic DCA data sets generated in one of N augmented manners, synthetic DCA data sets generated in two of N augmented manners, and so on, may be used. The percentages may be modified based on the type of input data, results achieved, and the like.

At 508, the reference and augmented DCA data sets are input into the convolutional layer C1 410 of the CNN model 400. As discussed previously, the reference and augmented DCA data sets can be a predetermined length long, such as 4s, between 4s and 10s, greater than 10s, etc. The length of each of the data set segments may vary, and thus is it not required that each segment be the same length.

At 510, the one or more processors utilize the augmented collection of reference and synthetic DCA data sets to train the CNN model 400 as discussed herein, evaluating the heart's rhythm without considering the device information (e.g., DD markers).

The trained CNN model 400 may provide various types of outputs. In one example, the CNN model 400 may be trained to output simply an indication of whether a corresponding DCA data set is valid or invalid.

Additionally or alternatively, the CNN model 400 may be trained to output a confidence indicator (e.g., a probability, likelihood, continuous value between 0 and 1) indicative of a level or degree of confidence that an individual underlying DCA data set represents a true positive or false positive designation of an arrhythmia (e.g., pause). As one example, the numeric indicator may be a continuous value between 0 and 1, where the values close to zero indicate a high confidence that a SEGM signal within the DCA data set is not indicative of pause, and thus a false positive. The values close to 1 indicate a high confidence that a SEGM signal within the DCA data set is indicative of pause and thus is a true positive. When a DCA data set is not indicative of pause, the DCA data set may include CA signals that are indicative of normal sinus rhythm or otherwise.

Additionally or alternatively, the CNN model 400 may be trained to provide outputs, in connection with each DCA data set, at least one of: i) a confidence indicator indicative of a degree of confidence that the corresponding DCA data set represents a true positive or false positive designation of pause, ii) a confidence indicator indicative of an accuracy of pause sensing implemented by the IMD, iii) a recommendation indicative of a sensitivity level to be utilized by the IMD to identify pause in the CA signals, iv) an indicator that the associated DCA data set is a true positive, v) an indicator that the associated DCA data set is a false positive, vi) an indicator that the associated DCA data set is a true negative, vii) an indicator that the associated DCA data set is a false negative, viii) uncertain/inconclusive DCA data set, or ix) invalid DCA data set. In some embodiments, the number of results in the uncertain/inconclusive category can be minimized by adding more samples to the training data set that are identified by the CNN as uncertain but using similarity, or by random sampling and increasing the training data set size. In other embodiments, if the event is infrequent, the event may not be evaluated by CNN due to infrequency of the event. In still further embodiments, DCA data sets with lower confidence metrics can be sent to a physician or other adjudicator for review.

Additionally or alternatively, the CNN model 400 may be trained to include a detection threshold that may be changed/tuned by clinicians based on the clinicians needs and various factors. In the foregoing example, where numeric values near 1 indicate a high confidence of true positives and numeric values near 0 indicate a high confidence of false positives, the detection threshold may be lowered (e.g., closer to 0) to increase the sensitivity of the CNN model 400. For example, when the detection threshold is set at .25, the CNN model 400 will identify more false positive DCA data sets, as compared to when the detection threshold is set at .75. For example, it may be desirable to apply a higher level of sensitivity in connection with certain types of critical arrhythmias (e.g., pause events) that may not occur regularly. In addition, it may be desirable to apply a higher threshold, and thus lower the sensitivity, while increasing the specificity, in connection with other types of pause events that are considered less "critical" to a patient’s health and that may occur more often.

At 512, the one or more processors save the CNN model 400 for subsequent use. For example, the CNN model 400 can be saved in an IMD 100, 212 that is or will be associated with a patient, an external instrument 154, 214, and/or one or more remote systems. The process of Figure 5 may be implemented across a population, medical network in another region to build one or more CNN models, etc. Additionally or alternatively, the CNN models can be custom-trained per clinic. The training can be done on a regular interval or as needed. Clinics can choose to train on their own data, or on "trusted" clinics’ data, or both. Performance across model versions can be compared, and clinics can optionally revert to earlier models.

Additionally or alternatively, the CNN model 400 may be constructed in alternative manners and/or utilize alternative parameters/hyperparameters. For example, the CNN model may utilize other activation function(s) such as leaky ReLu, Sigmoid, tanh, etc. Network architecture could be varying number of network layers, sub-layers, hidden layers, etc., other ways of performing data augmentation. Additionally or alternatively, the CNN model 400 may be trained to perform beat-level adjudication for greater visibility into classification behavior. Additionally or alternatively, the CNN model 400 may be trained to alert clinician to inappropriate sensing and/or recommend device reprogramming, changes to one or more settings, etc.

Once the main CNN model 400 is saved at 512, at 514, the CNN model 400 can be personalized, such as by combining additional features with the learned EGM features at the fully connected layer 406 to generate a personalized CNN model. Any number N personalized CNN models can be made to personalize healthcare and treatment for one or more patients.

Similarly, at 516 transfer learning can be used to train a new CNN model based on the CNN model 400 of 510 or the personalized CNN model of 514, as discussed previously.

The operations of Figure 5 may be staged to be performed upon the CA signals at various times, such as in real-time (e.g., during or shortly after a patient experiences an episode) or at any time after storage of the CA signals. The operations of Figure 5 may be performed by devices and systems at various proximity to a patient with the IMD 100. For example, the CA signals may be read out of an IMD and transmitted to a local portable external device (e.g., smartphone, table computer, laptop computer, smartwatch, etc.), where the local portable external device locally implements all or a portion of the operations described in connection with Figure 5 while in close proximity to the patient. In other embodiments, the CA signals can be transmitted/transferred to a remote device/system that implements all or a portion of the operations described in connection with Figure 5.

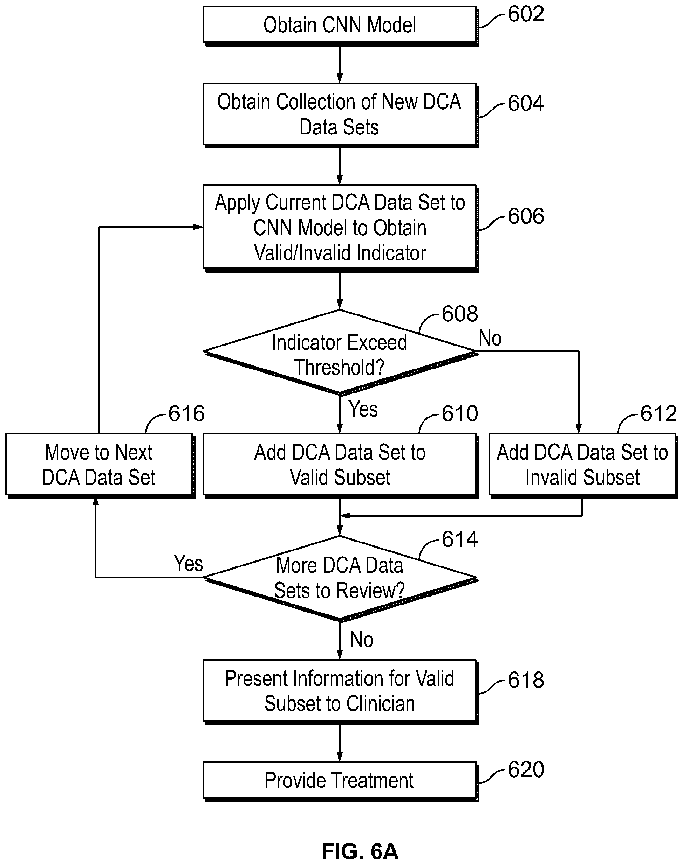

Figure 6A illustrates a process for discriminating between valid and invalid device classified pause episodes in accordance with embodiments herein. The operations of Figure 6A may be implemented by hardware, firmware, circuitry and/or one or more processors housed partially and/or entirely within an IMD, a local external device, remote server, a combination thereof, and/or more generally within a healthcare system. Optionally, the operations of Figure 6A may be partially implemented by an IMD and partially implemented by a local external device, remote server or more generally within a healthcare system. For example, the IMD includes IMD memory and one or more IMD processors, while each of the external devices/systems (ED) (e.g., local, remote or anywhere within the healthcare system) include ED memory and one or more ED processors.

At 602, the one or more processors of the system obtain one or more CNN models to be utilized in connection with analyzing previously acquired DCA data sets. For example, an external device may obtain the CNN model from a collection of CNN models, saved in the memory of the external device and/or at a remote server. The CNN model selected from the collection may be chosen based on the type of pause in the DCA data sets, a particular CNN model tuned for a specific patient or class of patients, etc. Additionally or alternatively, the CNN model may be selected based on the type or model of IMD, type or version of the pause detection algorithm utilized by the IMD and the like. Additionally or alternatively, the CNN model may be selected based upon the clinician or clinic that will be reviewing the DCA data sets, based upon characteristics of the patient (e.g., age, sex, weight, ethnic background, medical history, types of implanted devices, duration of device implant). In some embodiments, the same patient data may be analyzed by two or more CNN models.

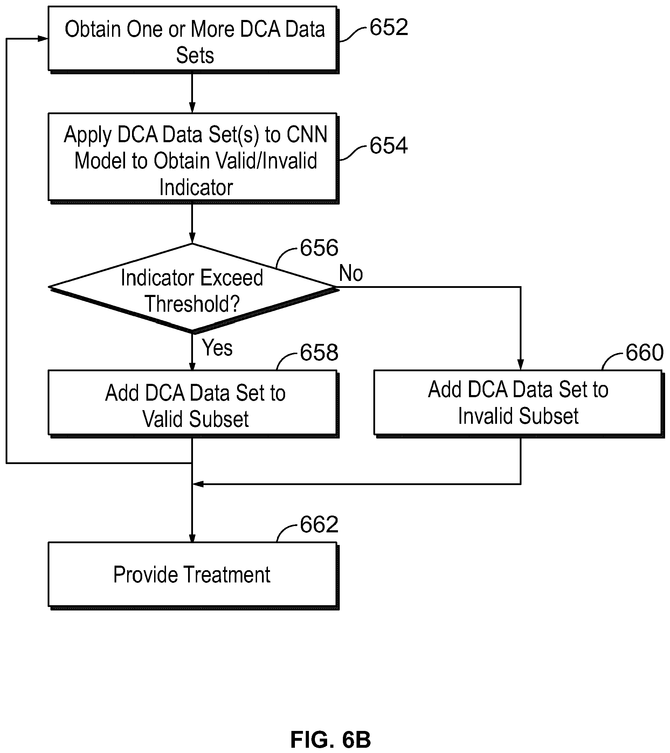

At 604, one or more processors of the system obtain a collection of DCA data sets generated by an IMD for a corresponding collection of candidate pause episodes declared by the IMD. The DCA data sets may also be referred to as "candidate" DCA data sets as the CNN model 400 has not yet verified the determination by the IMD that pause was in fact occurring. Each DCA data set may correspond to a separate candidate pause episode. Additionally or alternatively, a subset of the DCA data sets may correspond to related candidate pause episodes, such as when a patient is experiencing multiple related pause episodes that occur successively. The DCA data sets may be generated by the IMD over a period of time prior to implementation of the operations of Figure 6, and/or generated in real-time by the IMD during the operations of Figure 6. For example, an IMD may monitor a patient over several hours, days, weeks or longer and collect DCA data sets. Periodically, the previously acquired DCA data sets may be wirelessly transmitted (uploaded) from the IMD to an external device for storage and/or further transmission to a remote server. The obtaining operation at 604 may correspond simply to an external device and/or remote server accessing a previously stored collection of DCA data sets. Additionally or alternatively, the obtaining operation at 604 may at least partially include wireless reception of the DCA data sets at the external device and/or receipt of the DCA data sets at a remote server 304 (e.g., over a network).

Each of the DCA data sets includes far field cardiac activity (CA) signals for one or more beats sensed by an implantable medical device (IMD) and (optionally) one or more device documented (DD) markers that are generated by the IMD. For example, a single DCA data set corresponds to one pause episode and/or a series of pause episodes occurring successively in a short period of time. For example, one DCA data set may extend over a series of heartbeats (e.g., 7-10 beats). Additionally or alternatively, the DCA data set may be defined to include CA signals over a predetermined period of time (e.g., 30 s) preceding and/or following one or more pause episodes. Additionally or alternatively, the DCA data set can include approximately 60 seconds of an EGM, of which a window is selected, prior to the trigger for evaluation, that is of the duration that the CNN is expecting based on the CNN's design. An example of the trigger and the window are shown in Figure 4C.

In some embodiments, the CNN model 400 ignores the DD markers. The DD markers may be utilized by clinicians or other personnel when personally reviewing the DCA data sets, such as to confirm the diagnosis of the CNN model 400. In other embodiments, the IMD will forego generating the DD markers for data to be processed by the CNN model 400.

At 606, the one or more processors apply the DCA data sets to the CNN model 400 and in response thereto obtain a confidence indicator, valid/invalid indicator, etc., from the CNN model 400, indicative of a level or degree of confidence that the current DCA data set represents a true or false positive designation of pause. For example, the confidence indicator may represent a probability or likelihood that the corresponding DCA data set corresponds to pause or corresponds to a normal sinus rhythm. Additionally or alternatively, the confidence indicator may represent a numeric indicator between 0 and 1, where the values close to zero indicate a high confidence that a subcutaneous EGM signal within the DCA data set is not indicative of pause (e.g., a normal sinus rhythm, an unduly noisy signal that should not be otherwise characterized, an abnormal rhythm that is indicative of another condition other than pause), and thus a false positive. The values close to 1 indicate a high confidence that a subcutaneous EGM signal within the DCA data set is indicative of an arrhythmia and thus a true positive. The CNN model 400 may output, in connection with each DCA data set, at least one of: i) a confidence indicator indicative of a degree of confidence that the corresponding DCA data set represents a true positive or false positive designation of pause; ii) a confidence indicator indicative of an accuracy of pause sensing implemented by the IMD; or iii) a recommendation indicative of a sensitivity level to be utilized by the IMD to identify R waves in the CA signals. In some embodiments the confidence indicator levels can be set for different arrhythmia types. For example, the confidence indicator levels may be set in a first setting when indicating a first type of pause and in a second setting when indicating a second type of pause.

At 608, the one or more processors compare the confidence indicator provided by the CNN model 400 to one or more detection threshold. In the foregoing example, the confidence indicator may correspond to numeric values near 1 that indicate a high confidence of true positives, to numeric values near 0 that indicate a high confidence of false positives, and any value there between. The detection threshold may be changed/tuned by clinicians based on the clinicians needs and various factors. The detection threshold may be lowered (e.g., closer to 0) to increase the sensitivity of the CNN model. For example, when the detection threshold is set at .25, the CNN model will identify a larger number of false positive DCA data sets, as compared to when the detection threshold is set at .75. For example, it may be desirable to apply a higher level of sensitivity in connection with certain types of pause that may not occur regularly. In addition, it may be desirable to apply a higher threshold, and thus lower the sensitivity, while increasing the specificity, in connection with other types of pause that are considered less "critical" to a patient's health and that may occur more often. When the confidence indicator exceeds the threshold, flow moves to 610. When the confidence indicator does not exceed the threshold, flow moves to 612. In some embodiments, different detection thresholds can be set for different types of pause and/or different types of arrhythmias. Different detection thresholds can also be set by a practitioner, a clinic, etc.

At 610, the one or more processors add the DCA data set to a list of valid data sets to begin building a valid subset of DCA data sets. At 612, the one or more processors add the DCA data set to a list of invalid data sets to begin building an invalid subset of DCA data sets.

At 614, the one or more processors determine whether there are additional DCA data sets to be examined from the collection. If so, flow moves to 616, the one or more processors move to the next DCA data set within the collection and the operations at 606 - 614 are repeated. Otherwise, flow moves to 618.

The operations of Figure 6 are iteratively repeated until all of the DCA data sets have been analyzed by the CNN model 400 and grouped into the valid or invalid subset.

At 618, the one or more processors then present information to a clinician related to at least the valid subset of DCA data sets. The presented information may vary. For example, the information may display and allow the clinicians to analyze each of the DCA data sets in the valid subset. Additionally or alternatively, the information may include content related to the invalid subset. For example, the clinician may be informed that 80% of the DCA data sets were identified by the CNN model to be valid, while 20% were identified to be invalid. The information may include an option to allow the clinician to view the invalid subset of DCA data sets.

At 620, the one or more processors provide a treatment to the patient and/or provide a recommendation for a treatment to the patient. In some cases, the treatment and/or recommendation for a treatment can increase the DCA data sets identified as the valid subset of the DCA data sets. Additionally or alternatively, in response to the change in the treatment for the patient or the IMD, steps 604-620 may be iteratively repeated. In some embodiments, the one or more processors can confirm that the subsequent invalid subset of the DCA data sets has fewer invalid candidate pause episodes compared to the previous invalid subset. Further recommendations for treatment can iteratively be made based on the results of the comparison.

Additionally or alternatively, the treatment and/or information may include observations and/or recommendations regarding the threshold utilized in the process of Figure 6 to allow the clinician to adjust the level of sensitivity and/or specificity. For example, the clinician may be presented with the option to adjust the detection threshold. In response thereto, the process of Figure 6 may repeat at least the decision and subset classification at operations 608 - 614 to "reclassify" a portion of the invalid subset. For example, when the clinician is informed that 50% of the DCA data sets were initially identified to be invalid, the clinician may choose to adjust the threshold (e.g., changing from 0.6 to 0.75). With the adjusted threshold, the process may reclassify the invalid subset, for example thereafter informing the clinician that now only 20% of the DCA data sets are determined to be invalid. Additionally or alternatively, a numeric count of the number of DCA data sets may be provided instead of a percentage (e.g., the valid list includes 75 DCA data sets and the invalid list includes 13).

In some embodiments, the detection threshold may be automatically adjusted, such as within a predetermined range, to reduce the number of DCA data sets that were identified to be invalid (e.g., false positive) and the process may automatically reclassify the invalid subset.

Additionally or alternatively, the treatment and/or information may include observations or recommendations regarding potential adjustments that may be made to the parameters of the IMD to reduce the number of false positives. For example, the CNN model 400 may output an indication that the IMD may be performing inappropriate sensing, such as over sensing or under sensing P waves, R waves, T waves and the like. As a further indication, the CNN model 400 may identify an accuracy of the R wave sensing implemented by the IMD. Additionally or alternatively, the CNN model 400 may output recommendations for how to reprogram parameters of the IMD, such as how to reprogram sensitivity level/threshold utilized in connection with identifying P waves, R waves and T waves.

In some embodiments, sensing parameters of the IMD may be automatically adjusted, such as within a predetermined range, to modify sensing of one or more of P waves, R waves and T waves, to reduce the number of false positives. The one or more processors may evaluate the new DCA data sets to determine whether the automatically adjusted sensing parameters reduce the number of false positives.

Additionally or alternatively, the one or more processors can evaluate metrics such as true pause (e.g., true positive), false pause (e.g., false positive), true negative, false negative, uncertain/inconclusive, the confidence score, and/or other metrics discussed herein. The one or more processors can use these metrics to further edit the diagnosis, clear alerts (e.g., on the IMD, external instrument), and manage other pieces of the presentation, such as how urgent/important the event is based on whether the patient has been previously diagnosed and is on optimal medical treatment with no further interventions planned, or whether this is diagnosis and start of therapy based on additional status inputs from the providers. The initiation or adjustment of medications, therapies, patient actions, etc., can be suggested. Programmable changes to the settings of the IMD can be suggested, automatically implemented after acceptance by the practitioner, adjusted by the practitioner, and the like.

Optionally, the operations of Figures 5 and 6A may be repeated for different architectures of CNN models. For example, various aspects of the CNN model may be varied, such as the number of convolutional layers, hyperparameters, different activation functions, etc.

The operations of Figures 5 and 6A may be performed at regular intervals or as needed. Clinicians may choose to utilize their own DCA and DCNS data sets to train CNN models (which are augmented as described herein), such that the training data may be specific to a clinician, a single clinic, medical facility, medical network or otherwise. Additionally or alternatively, clinicians may choose to utilize a larger patient population of DCA and DCNS data sets to train the CNN models, such as by utilizing data sets collected by "trusted" third parties, or a combination thereof.

Optionally, the performance between CNN models trained or based on different augmented DCA data sets and/or different types/versions of models may be compared. For example, a clinician may apply the same augmented DCA data sets to a new version/type of CNN model that was previously used with the prior CNN model. The clinician may then be allowed to choose whether to use a newer version/types of CNN model based on whether the performance is better than the prior model.

While the foregoing examples related to Figure 6A are described in connection with analyzing DCA data sets related to pause episodes designated by an IMD, it is recognized that the operations of Figure 6A may be further applied to DCNS data sets collected by the IMD in connection with cardiac beats designated by the IMD to exhibit normal sinus rhythm. For example, one or more DCNS data sets may be analyzed separately from or in combination with the DCA data sets.

In the foregoing examples, the processors and memory are described to be housed within an implantable device, such as an ICM and/or IMD. Additionally or alternatively, the processors and memory may be housed within at least one of a local external device and a remote server.

Figure 6B illustrates a process for using the CNN model to determine and/or implement treatment for a patient in accordance with embodiments herein. The operations of Figure 6B may be implemented by hardware, firmware, circuitry and/or one or more processors housed partially and/or entirely within an IMD, a local external device, remote server, a combination thereof, and/or more generally within a healthcare system. Optionally, the operations of Figure 6B may be partially implemented by an IMD and partially implemented by a local external device, remote server or more generally within a healthcare system. For example, the IMD includes IMD memory and one or more IMD processors, while each of the external devices/systems (ED) (e.g., local, remote or anywhere within the healthcare system) include ED memory and one or more ED processors. It should be recognized that while the operations of Figure 6B are described in a somewhat serial manner, one or more of the operations may be continuous and/or performed in parallel with one another. For example, the various operations of the IMD 100, 212 may be continuous and/or performed in parallel with one another and/or other functions of the IMD 100, 212 and/or external device.

The process of Figure 6B is described primarily from the viewpoint of housing the CNN model within the IMD 100, 212. However, the CNN model can be housed within a local external device and/or within a device/system that is remote with respect to the patient.

At 652, one or more processors obtain one or more DCA data sets generated by an IMD that have been declared by the IMD as including candidate pause episodes. The DCA data sets may also be referred to as "candidate" DCA data sets as the CNN model 400 has not yet verified the determination by the IMD that pause was in fact occurring. Each DCA data set may correspond to a separate candidate pause episode. Additionally or alternatively, a subset of the DCA data sets may correspond to related candidate pause episodes, such as when a patient is experiencing multiple related pause episodes that occur successively. The DCA data sets may be generated by the IMD over a period of time before being applied to the CNN model or may be applied to the CNN model as soon as declared by the IMD (e.g., in real time or near real time). As described herein, each of the DCA data sets corresponds to one or more declared pause episodes, can be different lengths in time and include a series of heart beats, include or not include DD markers, etc.

Similar to 606 of Figure 6A, at 654, the one or more processors apply the DCA data set(s) to the CNN model 400 and in response thereto obtain a confidence indicator, valid/invalid indicator, etc., from the CNN model 400, indicative of a level or degree of confidence that the current DCA data set represents a true or false positive designation of pause. Also, for example, the CNN model 400 may output, in connection with each DCA data set, at least one of: i) a confidence indicator indicative of a degree of confidence that the corresponding DCA data set represents a true positive or false positive designation of pause; ii) a confidence indicator indicative of an accuracy of pause sensing implemented by the IMD; or iii) a recommendation indicative of a sensitivity level to be utilized by the IMD to identify R waves in the CA signals. In some embodiments the confidence indicator levels can be set for different arrhythmia types. For example, the confidence indicator levels may be set in a first setting when indicating a first type of pause and in a second setting when indicating a second type of pause.

Similar to 608 of Figure 6A, at 656 the one or more processors compare the confidence indicator(s) provided by the CNN model 400 to one or more detection threshold. In the foregoing example, the confidence indicator may correspond to numeric values near 1 that indicate a high confidence of true positives, to numeric values near 0 that indicate a high confidence of false positives, and any value there between. In some embodiments, when the confidence indicator exceeds the threshold, flow moves to 658 and the one or more processors add the DCA data set to a list of valid data sets to begin building a valid subset of DCA data sets. When the confidence indicator does not exceed the threshold, flow moves to 660 where the one or more processors add the DCA data set to a list of invalid data sets to begin building an invalid subset of DCA data sets. In some cases, valid and invalid subsets may not be collected.

The method can return to 652 to continue evaluation of the one or more DCA data sets as acquired/available.

At 662, the one or more processors provide a treatment to the patient. In some embodiments, the one or more processors may adjust or tune the detection threshold within predetermined limits to increase or decrease sensitivity as discussed herein based on the outputs of the CNN model. For example, the detection threshold may be adjusted to keep the percentage of DCA data sets indicated valid within predetermined limits. This provides the advantage of preventing over treating or under treating the patient. In other embodiments, the one or more processors may adjust, such as within predetermined limits, a sensitivity level used when sensing P waves, R waves, and/or T waves based on the outputs of the CNN model. This provides the advantage of preventing the over sensing or under sensing of the P waves, R waves, and/or T waves and reducing the number of false positives. In still further embodiments, the one or more processors may adjust therapy settings within predetermined limits based on the outputs of the CNN model. For example, sensing parameters of the IMD may be adjusted, pacing may be adjusted or temporarily suspended, etc.

662 can be repeated as data is output by the CNN model over time. A record of any adjustments, and optionally the DCA data sets, valid subsets, and/or invalid subsets, can be retained in a memory by the IMD and/or external device until they are transmitted to a healthcare provider for review. Treating pause provides the advantage of quickly addressing medical issues and/or changes with a patient. By setting predetermined limits, there is no need to wait for a clinician to review and reprogram the patient's IMD. In some embodiments, the IMD and/or external device can send a message, such as to a remote system, to indicate that settings have been adjusted. In other embodiments, a message can be sent if adjustments beyond the predetermined limits appear warranted.

Figure 7 illustrates a distributed processing system 700 in accordance with embodiments herein. The distributed processing system 700 includes a server 702 connected to a database 704, a programmer 706, a local monitoring device 708 (e.g., cell phone, tablet, computer, other medical monitoring device) and a user workstation 710 electrically connected to a network 712. Any of the processor-based components in Figure 7 (e.g., IMD 703, workstation 710, cell phone 714, local monitoring device 716, server 702, programmer 706) may perform the processes discussed herein.

The network 712 may provide cloud-based services over the internet, a voice over IP (VoIP) gateway, a local plain old telephone service (POTS), a public switched telephone network (PSTN), a cellular phone-based network, and the like. Alternatively, the communication system may be a local area network (LAN), a medical campus area network (CAN), a metropolitan area network (MAN), or a wide area network (WAM). The communication system serves to provide a network that facilitates the transfer/receipt of data and other information between local and remote devices (relative to a patient). The server 702 is a computer system that provides services to the other computing devices on the network 712. The server 702 can control the communication of information such as CNN models, DCA data sets, synthetic DCA data sets, CA signals, motion data, pause episode information, bradycardia episode information, arrythmia episode information, markers, heart rates, and device settings. The server 702 interfaces with the network 712 to transfer information between the programmer 706, local monitoring devices 708, 716, user workstation 710, cell phone 714 and database 704. For example, the server 702 may receive DCA data sets from various clinics, medical networks, individual patients and the like and utilize the DCA data sets and synthetic DCA data sets to train new CNN models, update existing versions of CNN models, personalize CNN models, apply transfer learning to CNN modules, and add further outputs to existing and new CNN models. The server 702 may further push new CNN models, updated versions of CNN models, and/or personalized CNN models to various other devices, such as the programmers, local monitoring devices, cell phones, workstations and the like illustrated in Figure 7. The database 704 stores information such as DCA data sets, synthetic DCA data sets, pause episode information, arrythmia episode information, pause statistics, arrythmia statistics, diagnostics, DD markers, CA signals, heart rates, device settings, and the like, for a patient population, as well as separately for individual patients, individual physicians, individual clinics, individual medical networks and the like. The server 702 may implement the CNN training operations described in connection with Figure 5 and/or utilize the CNN models to analyze the DCA data sets as described in connection with Figures 6A and 6B. The database 704 also maintains the CNN models trained, updated, and personalized as described herein. The CNN models and other information are downloaded into the database 704 via the server 702 or, alternatively, the information is uploaded to the server 702 from the database 704. The programmer 706 may reside in a patient's home, a hospital, or a physician's office. The programmer 706 may wirelessly communicate with the ICM or IMD 703 and utilize protocols, such as Bluetooth, GSM, infrared wireless LANs, HIPERLAN, 3G, satellite, as well as circuit and packet data protocols, and the like. Alternatively, a telemetry "wand" connection may be used to connect the programmer 706 to the IMD 703. The programmer 706 is able to acquire cardiac signals from surface ECG electrodes 722 on a person (e.g., ECGs), electrograms (e.g., EGM) signals from the IMD 703, and/or CA data, pause episode information, arrythmia episode information, pause statistics, arrythmia statistics, diagnostics, markers, CA signal waveforms, device settings from the IMD 703. The programmer 706 interfaces with the network 712, such as via the internet, to upload the information acquired from the surface ECG unit 720, or the IMD 703 to the server 702.

The local monitoring device 708 interfaces with the communication system to upload to the server 702 one or more of the DCA data sets, synthetic DCA data sets, CA signals, motion data, pause episode information, arrythmia episode information, pause statistics, arrythmia statistics, diagnostics, markers, heart rates, sensitivity profile parameter settings and detection thresholds. In one embodiment, the surface ECG unit 720 and the IMD 703 have a bi-directional connection 724 with the local RF monitoring device 708 via a wireless connection. The local monitoring device 708 is able to acquire surface ECG signals from an ECG lead 722, as well as DCA CA data sets and other information from the IMD 703. On the other hand, the local monitoring device 708 may download the data and information discussed herein, such as CNN models, from the database 704 to the IMD 703.

The user workstation 710, cell phone 714 and/or programmer 706 may be utilized by a physician or medical personnel to interface with the network 712 to download CNN models, DCA data sets, synthetic DCA data sets, CA signals, motion data, and other information discussed herein from the database 704, from the local monitoring devices 708, 716, from the IMD 703 or otherwise. Once downloaded, the user workstation 710 may process the DCA data sets, CA signals and motion data in accordance with one or more of the operations described above. The user workstation 710, cell phone 714 and/or programmer 706 may be used to present information, via display, printing, etc., concerning at least one of the valid subset or invalid subset of the DCA data sets to a physician, present information concerning suggested adjustments to device settings, history of automatically implemented adjustments to device settings, and the like. Additionally or alternatively, the user workstation 710, cell phone 714 and/or programmer 706 may be utilized to display, in connection with each DCA data set, at least one of: i) a confidence indicator indicative of a degree of confidence that the corresponding DCA data set represents a true positive or false positive designation of pause; ii) a confidence indicator indicative of an accuracy of pause sensing implemented by the IMD; or iii) a recommendation indicative of a sensitivity level to be utilized by the IMD to identify R waves in the CA signals.

The user workstation 710, cell phone 714 and/or programmer 706 may upload/push settings (e.g., sensitivity profile parameter settings), IMD instructions, other information and notifications to the cell phone 714, local monitoring devices 708, 716, programmer 706, server 702 and/or IMD 703.

The processes described herein in connection with reducing false positive pause events and/or determining and implementing treatment may be performed by one or more of the devices illustrated in Figure 7, including but not limited to the IMD 703, programmer 706, local monitoring devices 708, 716, user workstation 710, cell phone 714, and server 702. The process described herein may be distributed between the devices of Figure 7. For example, one or more of the devices illustrated in Figure 7 may include memory to store specific executable instructions and one or more CNN model trained to detect pause episodes that comprises a GAP layer; and one or more processors configured to execute the specific executable instructions to: obtain device classified arrhythmia (DCA) data sets generated by an implantable medical device (IMD) for corresponding candidate pause episodes declared by the IMD, the DCA data sets including cardiac activity (CA) signals for one or more beats sensed by the IMD; and apply the CNN model to the DCA data sets to identify a valid subset of the DCA data sets that correctly characterizes the corresponding CA signals. One or more of the devices illustrated in Figure 7 may further include a display configured to present information concerning the valid subset of the DCA data sets.

Additionally or alternatively, wherein the one or more processors are further configured to identify an invalid subset of the DCA data sets that incorrectly characterizes the corresponding CA signals, wherein the valid subset of the DCA data sets indicates a portion of the DCA data sets that indicates true pause, and the invalid subset of the DCA data sets indicates a second portion of the DCA data sets that indicates false pause. Additionally or alternatively, the information is indicative of a recommendation for i) a change in a treatment for a patient associated with the IMD that will increase the DCA data sets identified as the valid subset of the DCA data sets, or ii) a change in a treatment associated with the IMD that will increase the DCA data sets identified as the valid subset of the DCA data sets. Additionally or alternatively, wherein the one or more processors are further configured to apply the CNN model to the DCA data sets to identify an invalid subset of the DCA data sets that incorrectly characterizes the corresponding CA signals, wherein in response to the change in the treatment for the patient or the IMD, the one or more processors are further configured to obtain second DCA data sets generated by the IMD, apply the CNN model to the second DCA data sets to identify a second invalid subset of the DCA data sets that incorrectly characterizes the corresponding CA signals, and confirm that the second invalid subset of the DCA data sets has fewer invalid candidate pause episodes compared to the invalid subset of the DCA data sets.

Additionally or alternatively, the CNN model comprises at least two 1-dimensional (1D) convolutional layers, the GAP layer configured to receive outputs from each of the at least two 1D convolutional layers. Additionally or alternatively, the CNN model comprises five 1D convolutional layers. Additionally or alternatively, the at least two 1D convolutional layers automatically capture different levels of features associated with pause from input data associated with the DCA data sets.

Additionally or alternatively, the one or more processors may be further configured to process on output of a first 1D convolutional layer using i) a rectified linear unit activation function, ii) a batch normalization function, or iii) a dropout function. Additionally or alternatively, the CNN model comprises at least first and second 1D convolutional layers, and the one or more processors may be further configured to: normalize an output of the first 1D convolutional layer and send the normalized output of the first 1D convolutional layer to the second 1D convolutional layer. Additionally or alternatively, the CNN model further comprises a fully connected layer configured to receive input from the GAP layer. Additionally or alternatively, the CNN model outputs, in connection with each of a plurality of the DCA data sets, a confidence indicator indicative of a degree of confidence that the corresponding DCA data set represents a true positive or false positive designation of pause. Additionally or alternatively, the one or more processors are further configured to compare the confidence indicator to a detection threshold, wherein the information further comprises information concerning the valid subset of the DCA data sets that exceed the detection threshold. Additionally or alternatively, in response to an adjustment to the detection threshold, the one or more processors are further configured to present the information concerning the valid subset of the DCA data sets that exceed the adjusted detection threshold. Additionally or alternatively, the CNN model further comprises a Softmax layer configured to output the confidence indicator. Additionally or alternatively, the CNN model represents a model that is trained utilizing an augmented collection of DCA data sets, wherein the augmented collection of the DCA data sets includes reference DCA data sets from patients and synthetic DCA data sets that are generated based on the reference DCA data sets. Additionally or alternatively, wherein the synthetic DCA data sets are generated using i) noise addition, ii) signal inversion, iii) magnification, iv) inserting one or more p waves during a pause interval at a previous R-R interval, v) stretching or shrinking a duration of a pause interval, or vi) two or more of noise addition, signal inversion, magnification, inserting one or more p waves during a pause interval at a previous R-R interval, or stretching or shrinking a duration of a pause interval. Additionally or alternatively, wherein the information is indicative of a recommendation for adjustment to a sensing parameter or a therapy parameter associated with the IMD. Additionally or alternatively, the system further including the IMD that comprises a combination of subcutaneous electrodes configured to collect the CA signals, IMD memory configured to store program instructions, and one or more IMD processors configured to execute the program instructions to analyze the CA signals and based on the analysis declare candidate pause episodes and generate the DCA data sets including the corresponding CA signals; and system also including a transceiver configured to wirelessly transmit the DCA data sets to an external device. Additionally or alternatively, the system of Figure 7 further comprises an external device that includes the memory and the one or more processors and a transceiver, the transceiver configured to wirelessly receive the DCA data sets from the IMD. Additionally or alternatively, the system of Figure 7 further comprises a server that includes the memory and the one or more processors, the memory configured to store a collection of the DCA data sets, the one or more processors configured to apply the CNN model to the collection of the DCA data sets.

The system of Figure 7 further comprises, under control of one or more processors configured with specific executable instructions, a method for building a convolutional neural network (CNN) model to confirm device documented (DD) pause episodes, comprising obtaining a collection of reference device classified arrhythmia (DCA) data sets associated with device declared pause episodes, the reference DCA data sets including cardiac activity (CA) signals for one or more beats sensed by electrodes of an implantable medical device (IMD); generating synthetic DCA data sets based on the reference DCA data sets to form an augmented collection of DCA data sets, wherein the generating includes processing the reference DCA data sets using i) noise addition or ii) signal inversion; and applying the DCA data sets and the augmented collection of DCA data sets to the CNN model to train the CNN model. In other embodiments the generating can include magnification, inserting one or more p waves during a pause interval at a previous R-R interval, and/or stretching or shrinking a duration of a pause interval, and/or other augmentation processes discussed herein and/or known in the art.

Additionally or alternatively, one or more processors of the system are further configured to apply a first augmented collection of the DCA data sets that represent valid DCA data sets that that correctly characterize the corresponding CA signals, and apply a second augmented collection of the DCA data sets that represent invalid DCA data sets that incorrectly characterize the corresponding CA signals. Additionally or alternatively, one or more processors of the system are further configured to automatically capture, using at least two convolutional layers, different levels of features associated with pause from input data associated with the DCA data sets and the augmented collection of DCA data sets. Additionally or alternatively, one or more processors of the system are further configured to receive, with a global average pooling (GAP) layer, outputs from at least two 1D convolutional layers. Additionally or alternatively, one or more processors of the system are further configured to tune the CNN model by inputting, into a layer of the CNN model, at least one of i) age-related feature, ii) gender, iii) body mass index, iv) ethnicity, v) medical history, vi) medication usage, vii) lifestyle factor, or viii) family history. Additionally or alternatively, one or more processors of the system are further configured to freeze at least one layer of the CNN model, and update, using additional DCA data sets, at least one layer of the CNN model that is not frozen.

In accordance with new and unique aspects herein, the IMD 100, 212, 703, cell phone 714, local monitoring device 708, programmer 706, server 702, and/or other processor(s) and/or processing system delivers a particular treatment for the medical condition of pause (e.g., pause episode(s)) based on outputs of the CNN model. The pause events detected by the IMD are further evaluated by the CNN model and true pause events are separated from false pause events. The treatment can be based on an indicator threshold, on what percentage of potential pause events are determined to be true pause, used to recommend setting changes in the IMD, used to recommend actions and/or behaviors of the patient and/or clinician, etc. For example, the clinician uses the true pause and/or false pause, along with any other outputs of the CNN model 400, to prescribe/change the patient's therapy (e.g., prescribe new medication, change medication, change diet, modify behavior, recommend implantation of another and/or a different IMD, change programmed parameters of the IMD already implanted). In another example, the IMD can automatically implement changes to programmed parameters of the IMD based on outputs of the CNN model. Thus, the methods and systems herein provide a practical application for treating the medical condition of pause in a patient.

Figure 8 illustrates a system level diagram indicating potential devices and networks that utilize the methods and systems herein. For example, an IMD 802 may be utilized to collect a DCA data set. The IMD 802 may supply the DCA data set (CA signals, DD markers) as well as sensitivity levels and optionally motion data, to various local external devices, such as a tablet device 804, a smart phone 806, a bedside monitoring device 808, a smart watch and the like. The devices 804-808 include a display to present the various types of the CA signals, DD markers, statistics (e.g., percentage or number valid, percentage or number invalid), diagnostics, recommendations for adjustments in IMD sensing/therapy parameters and other information described herein, and/or summary or list of automatic adjustments made in IMD sensing/therapy parameters. The IMD 802 may convey the DCA data sets over various types of wireless communications links to the devices 804, 806 and 808. The IMD 802 may utilize various communications protocols and be activated in various manners, such as through a Bluetooth, Bluetooth low energy, Wi-Fi or other wireless protocol. Additionally or alternatively, when a magnetic device 810 is held next to the patient, the magnetic field from the device 810 may activate the IMD 802 to transmit the DCA data set and arrythmia data to one or more of the devices 804-808.

In accordance with new and unique aspects herein, methods and systems are described to train and utilize machine learning models, such as CNN models, to determine whether candidate arrhythmias (e.g., pause), declared and classified by an IMD from subcutaneous EGMs (SEGMs), are true or false positives. Initial performance evaluation suggests that the CNN model may reduce the false positive burden, related to IMD declared pause, by improving sensitivity and specificity, as well as improving positive predictive value metrics.

Embodiments may be implemented utilizing all or portions of the methods and systems described in U.S. Patent Application 2022/0117538, filed June 8, 2021, entitled "Methods and systems to confirm device classified arrhythmias utilizing machine learning models" and U.S. Patent Application 2022/0304612, filed December 17, 2023, entitled "Methods and systems for predicting arrhythmia risk utilizing machine learning models".

Embodiments may be implemented in connection with one or more implantable medical devices (IMDs). Non-limiting examples of IMDs include one or more of implantable leadless monitoring and/or therapy devices, and/or alternative implantable medical devices. For example, the IMD may represent a cardiac monitoring device, pacemaker, cardioverter, cardiac rhythm management device, defibrillator, leadless monitoring device, leadless pacemaker and the like. Additionally or alternatively, the IMD may be a leadless implantable medical device (LIMD) that include one or more structural and/or functional aspects of the device(s) described in U.S. Patent 9,216,285 "LEADLESS IMPLANTABLE MEDICAL DEVICE HAVING REMOVABLE AND FIXED COMPONENTS" and U.S. Patent 8,831,747 "LEADLESS NEUROSTIMULATION DEVICE AND METHOD INCLUDING THE SAME". Additionally or alternatively, the IMD may include one or more structural and/or functional aspects of the device(s) described in U.S. Patent 8,391,980 "METHOD AND SYSTEM FOR IDENTIFYING A POTENTIAL LEAD FAILURE IN AN IMPLANTABLE MEDICAL DEVICE" and U.S. Patent 9,232,485 "SYSTEM AND METHOD FOR SELECTIVELY COMMUNICATING WITH AN IMPLANTABLE MEDICAL DEVICE". Additionally or alternatively, the IMD may be a subcutaneous IMD that includes one or more structural and/or functional aspects of the device(s) described in U.S. Patent No. 10,765,860, titled "SUBCUTANEOUS IMPLANTATION MEDICAL DEVICE WITH MULTIPLE PARASTERNAL-ANTERIOR ELECTRODES" and filed May 7, 2018; U.S. Patent No. 10,722,704, titled "IMPLANTABLE MEDICAL SYSTEMS AND METHODS INCLUDING PULSE GENERATORS AND LEADS" filed May 7, 2018; US Patent 11,045,643, titled "SINGLE SITE IMPLANTATION METHODS FOR MEDICAL DEVICES HAVING MULTIPLE LEADS", filed May 7, 2018. Additionally or alternatively, the IMD may be a leadless cardiac monitor (ICM) that includes one or more structural and/or functional aspects of the device(s) described in U.S. Patent 9,949,660, issued April 24, 2018, entitled, "METHOD AND SYSTEM TO DISCRIMINATE RHYTHM PATTERNS IN CARDIAC ACTIVITY"; U.S. Patent 10,729,346, titled "METHOD AND SYSTEM FOR SECOND PASS CONFIRMATION OF DETECTED CARDIAC ARRHYTHMIC PATTERNS"; U.S. Patent 11,020,036, titled "METHOD AND SYSTEM TO DETECT R-WAVES IN CARDIAC ARRHYTHMIC PATTERNS"; U.S. Patent 10,874,322, titled "METHOD AND SYSTEM TO DETECT PREMATURE VENTRICULAR CONTRACTIONS IN CARDIAC ACTIVITY SIGNALS"; U.S. Patent 10,777,880, titled "ADJUSTABLE ANTENNA SYSTEM TO COMMUNICATE WITH AN IMPLANTABLE MEDICAL DEVICE AND METHOD FOR USING SAME"; and U.S. Published Application 2021/0020294, filed July 16, 2020, titled "METHODS, DEVICES AND SYSTEMS FOR HOLISTIC INTEGRATED HEALTHCARE PATIENT MANAGEMENT". Further, one or more combinations of IMDs may be utilized from the above mentioned patents and applications in accordance with embodiments herein.

CLOSING

The various methods as illustrated in the Figures and described herein represent exemplary embodiments of methods. The methods may be implemented in software, hardware, or a combination thereof. In various of the methods, the order of the steps may be changed, and various elements may be added, reordered, combined, omitted, modified, etc. Various of the steps may be performed automatically (e.g., without being directly prompted by user input) and/or programmatically (e.g., according to program instructions).

Various modifications and changes may be made as would be obvious to a person skilled in the art having the benefit of this disclosure. It is intended to embrace all such modifications and changes and, accordingly, the above description is to be regarded in an illustrative rather than a restrictive sense.

Various embodiments of the present disclosure utilize at least one network that would be familiar to those skilled in the art for supporting communications using any of a variety of commercially-available protocols, such as Transmission Control Protocol/Internet Protocol ("TCP/IP"), User Datagram Protocol ("UDP"), protocols operating in various layers of the Open System Interconnection ("OSI") model, File Transfer Protocol ("FTP"), Universal Plug and Play ("UpnP"), Network File System ("NFS"), Common Internet File System ("CIFS") and AppleTalk. The network can be, for example, a local area network, a wide-area network, a virtual private network, the Internet, an intranet, an extranet, a public switched telephone network, an infrared network, a wireless network, a satellite network and any combination thereof.

In embodiments utilizing a web server, the web server can run any of a variety of server or mid-tier applications, including Hypertext Transfer Protocol ("HTTP") servers, FTP servers, Common Gateway Interface ("CGI") servers, data servers, Java servers, Apache servers and business application servers. The server(s) also may be capable of executing programs or scripts in response to requests from user devices, such as by executing one or more web applications that may be implemented as one or more scripts or programs written in any programming language, such as Java®, C, C# or C++, or any scripting language, such as Ruby, PHP, Perl, Python or TCL, as well as combinations thereof. The server(s) may also include database servers, including without limitation those commercially available from Oracle®, Microsoft®, Sybase®, SAS® and IBM® as well as open-source servers such as MySQL, Postgres, SQLite, MongoDB, and any other server capable of storing, retrieving and accessing structured or unstructured data. Database servers may include table-based servers, document-based servers, unstructured servers, relational servers, non-relational servers or combinations of these and/or other database servers.

The environment can include a variety of data stores and other memory and storage media as discussed above. These can reside in a variety of locations, such as on a storage medium local to (and/or resident in) one or more of the computers or remote from any or all of the computers across the network. In a particular set of embodiments, the information may reside in a storage-area network ("SAN") familiar to those skilled in the art. Similarly, any necessary files for performing the functions attributed to the computers, servers or other network devices may be stored locally and/or remotely, as appropriate. Where a system includes computerized devices, each such device can include hardware elements that may be electrically coupled via a bus, the elements including, for example, at least one central processing unit ("CPU" or "processor"), at least one input device (e.g., a mouse, keyboard, controller, touch screen or keypad) and at least one output device (e.g., a display device, printer or speaker). Such a system may also include one or more storage devices, such as disk drives, optical storage devices and solid-state storage devices such as random access memory ("RAM") or read-only memory ("ROM"), as well as removable media devices, memory cards, flash cards, etc.

Such devices also can include a computer-readable storage media reader, a communications device (e.g., a modem, a network card (wireless or wired), an infrared communication device, etc.) and working memory as described above. The computer-readable storage media reader can be connected with, or configured to receive, a computer-readable storage medium, representing remote, local, fixed and/or removable storage devices as well as storage media for temporarily and/or more permanently containing, storing, transmitting and retrieving computer-readable information. The system and various devices also typically will include a number of software applications, modules, services or other elements located within at least one working memory device, including an operating system and application programs, such as a client application or web browser. It should be appreciated that alternate embodiments may have numerous variations from that described above. For example, customized hardware might also be used and/or particular elements might be implemented in hardware, software (including portable software, such as applets) or both. Further, connection to other computing devices such as network input/output devices may be employed.

Various embodiments may further include receiving, sending, or storing instructions and/or data implemented in accordance with the foregoing description upon a computer-readable medium. Storage media and computer readable media for containing code, or portions of code, can include any appropriate media known or used in the art, including storage media and communication media, such as, but not limited to, volatile and non-volatile, removable and non-removable media implemented in any method or technology for storage and/or transmission of information such as computer readable instructions, data structures, program modules or other data, including RAM, ROM, Electrically Erasable Programmable Read-Only Memory ("EEPROM"), flash memory or other memory technology, Compact Disc Read-Only Memory ("CD-ROM"), digital versatile disk (DVD) or other optical storage, magnetic cassettes, magnetic tape, magnetic disk storage or other magnetic storage devices or any other medium which can be used to store the desired information and which can be accessed by the system device. Based on the disclosure and teachings provided herein, a person of ordinary skill in the art will appreciate other ways and/or methods to implement the various embodiments.

The units/modules/applications herein may include any processor-based or microprocessor-based system including systems using microcontrollers, reduced instruction set computers (RISC), application specific integrated circuits (ASICs), field-programmable gate arrays (FPGAs), logic circuits, and any other circuit or processor capable of executing the functions described herein. Additionally, or alternatively, the modules/controllers herein may represent circuit modules that may be implemented as hardware with associated instructions (for example, software stored on a tangible and non-transitory computer readable storage medium, such as a computer hard drive, ROM, RAM, or the like) that perform the operations described herein. The above examples are exemplary only, and are thus not intended to limit in any way the definition and/or meaning of the term "controller." The units/modules/applications herein may execute a set of instructions that are stored in one or more storage elements, in order to process data. The storage elements may also store data or other information as desired or needed. The storage element may be in the form of an information source or a physical memory element within the modules/controllers herein. The set of instructions may include various commands that instruct the modules/applications herein to perform specific operations such as the methods and processes of the various embodiments of the subject matter described herein. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to user commands, or in response to results of previous processing, or in response to a request made by another processing machine.

The specification and drawings are, accordingly, to be regarded in an illustrative rather than a restrictive sense. It will, however, be evident that various modifications and changes may be made thereunto without departing from the scope of the invention as set forth in the claims.

Other variations are within the scope of the present disclosure. Thus, while the disclosed techniques are susceptible to various modifications and alternative constructions, certain illustrated embodiments thereof are shown in the drawings and have been described above in detail. It should be understood, however, that there is no intention to limit the invention to the specific form or forms disclosed, but on the contrary, the intention is to cover all modifications, alternative constructions and equivalents falling within the spirit and scope of the invention, as defined in the appended claims.

The use of the terms "a" and "an" and "the" and similar referents in the context of describing the disclosed embodiments (especially in the context of the following claims) are to be construed to cover both the singular and the plural, unless otherwise indicated herein or clearly contradicted by context. The terms "comprising," "having," "including" and "containing" are to be construed as openended terms (i.e., meaning "including, but not limited to,") unless otherwise noted. The term "connected," when unmodified and referring to physical connections, is to be construed as partly or wholly contained within, attached to or joined together, even if there is something intervening. Recitation of ranges of values herein are merely intended to serve as a shorthand method of referring individually to each separate value falling within the range, unless otherwise indicated herein and each separate value is incorporated into the specification as if it were individually recited herein. The use of the term "set" (e.g., "a set of items") or "subset" unless otherwise noted or contradicted by context, is to be construed as a nonempty collection comprising one or more members. Further, unless otherwise noted or contradicted by context, the term "subset" of a corresponding set does not necessarily denote a proper subset of the corresponding set, but the subset and the corresponding set may be equal.

Operations of processes described herein can be performed in any suitable order unless otherwise indicated herein or otherwise clearly contradicted by context. Processes described herein (or variations and/or combinations thereof) may be performed under the control of one or more computer systems configured with executable instructions and may be implemented as code (e.g., executable instructions, one or more computer programs or one or more applications) executing collectively on one or more processors, by hardware or combinations thereof. The code may be stored on a computer-readable storage medium, for example, in the form of a computer program comprising a plurality of instructions executable by one or more processors. The computer-readable storage medium may be non-transitory.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the invention without departing from its scope. While the dimensions, types of materials and physical characteristics described herein are intended to define the parameters of the invention, they are by no means limiting and are exemplary embodiments. Many other embodiments will be apparent to those of skill in the art upon reviewing the above description. The scope of the invention should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc., are used merely as labels, and are not intended to impose numerical requirements on their objects

**Claims**

**1.** A system (200, 700) for declaring pause in cardiac activity, comprising:

memory (704) to store specific executable instructions and a convolutional neural network (CNN) model trained to detect pause episodes, the CNN model comprising a global average pooling (GAP) layer;
one or more processors (702) configured to execute the specific executable instructions to:

obtain device classified arrhythmia (DCA) data sets generated by an implantable medical device (IMD) (212, 703) for corresponding candidate pause episodes declared by the IMD (212, 703), the DCA data sets including cardiac activity (CA) signals for one or more beats sensed by the IMD (212, 703); and
apply the CNN model to the DCA data sets to identify a valid subset of the DCA data sets that correctly characterizes the corresponding CA signals; and

a display configured to present information concerning the valid subset of the DCA data sets.

**2.** The system of claim 1, wherein the one or more processors (702) are further configured to apply the CNN model to the DCA data sets to identify an invalid subset of the DCA data sets that incorrectly characterizes the corresponding CA signals, wherein the valid subset of the DCA data sets indicates a portion of the DCA data sets that indicates true pause, and the invalid subset of the DCA data sets indicates a second portion of the DCA data sets that indicates false pause.

**3.** The system of claim 1 or 2, wherein the information is indicative of a recommendation for i) a change in a treatment for a patient (208) associated with the IMD (212, 703) that will increase the DCA data sets identified as the valid subset of the DCA data sets, or ii) a change in a treatment associated with the IMD (212, 703) that will increase the DCA data sets identified as the valid subset of the DCA data sets.

**4.** The system of claim 3, wherein the one or more processors (702) are further configured to apply the CNN model to the DCA data sets to identify an invalid subset of the DCA data sets that incorrectly characterizes the corresponding CA signals, wherein in response to the change in the treatment for the patient (208) or the IMD (212, 703), the one or more processors (702) are further configured to:

obtain second DCA data sets generated by the IMD (212, 703);
apply the CNN model to the second DCA data sets to identify a second invalid subset of the DCA data sets that incorrectly characterizes the corresponding CA signals; and
confirm that the second invalid subset of the DCA data sets has fewer invalid candidate pause episodes compared to the invalid subset of the DCA data sets.

**5.** The system of any one of claims 1 to 4, wherein the CNN model outputs, in connection with each of a plurality of the DCA data sets, a confidence indicator indicative of a degree of confidence that the corresponding DCA data set represents a true positive or false positive designation of pause.

**6.** The system of claim 5, wherein the one or more processors (702) are further configured to compare the confidence indicator to a detection threshold, wherein the information further comprises information concerning the valid subset of the DCA data sets that exceed the detection threshold.

**7.** The system of claim 6, in response to an adjustment to the detection threshold, the one or more processors (702) are further configured to present the information concerning the valid subset of the DCA data sets that exceed the adjusted detection threshold.

**8.** The system of any one of claims 1 to 7, wherein the CNN model represents a model that is trained utilizing an augmented collection of DCA data sets, wherein the augmented collection of the DCA data sets includes reference DCA data sets from patients (208) and synthetic DCA data sets that are generated based on the reference DCA data sets.

**9.** The system of claim 8, wherein the synthetic DCA data sets are generated using i) noise addition, ii) signal inversion, iii) magnification, iv) inserting one or more p waves during a pause interval at a previous R-R interval, v) stretching or shrinking a duration of a pause interval, or vi) two or more of noise addition, signal inversion, magnification, inserting one or more p waves during a pause interval at a previous R-R interval, or stretching or shrinking a duration of a pause interval.

**10.** A computer implemented method to confirm device documented (DD) pause episodes, comprising:
under control of one or more processors (702) configured with specific executable instructions,

obtaining device classified arrhythmia (DCA) data sets generated by an implantable medical device (IMD) (212, 703) for corresponding candidate pause episodes declared by the IMD (212, 703), the DCA data sets including cardiac activity (CA) signals for one or more beats sensed by the IMD (212, 703);
applying a convolutional neural network (CNN) model trained to detect pause episodes to the DCA data sets to identify a valid subset of the DCA data sets that correctly characterizes the corresponding CA signals; and
presenting information concerning the valid subset of the DCA data sets.

**11.** The method of claim 10, further comprising applying the CNN model to the DCA data sets to identify an invalid subset of the DCA data sets that incorrectly characterizes the corresponding CA signals, wherein the valid subset of the DCA data sets indicates a portion of the DCA data sets that indicates true pause, and the invalid subset of the DCA data sets indicates a second portion of the DCA data sets that indicates false pause.

**12.** The method of claim 10 or 11, wherein the information is indicative of a recommendation for i) a change in a treatment for a patient (208) associated with the IMD (212, 703) that will increase the DCA data sets identified as the valid subset of the DCA data sets, or ii) a change in a treatment associated with the IMD (212, 703) that will increase the DCA data sets identified as the valid subset of the DCA data sets.

**13.** The method of claim 12, further comprising:

applying the CNN model to the DCA data sets to identify an invalid subset of the DCA data sets that incorrectly characterizes the corresponding CA signals; and
in response to the change in the treatment for the patient (208) or the IMD (212, 703), the method further comprises:

obtaining second DCA data sets generated by the IMD (212, 703);
applying the CNN model to the second DCA data sets to identify a second valid subset of the DCA data sets that correctly characterizes the corresponding CA signals and to identify a second invalid subset of the DCA data sets that incorrectly characterizes the corresponding CA signals; and
confirming that the second invalid subset of the DCA data sets has fewer invalid candidate pause episodes compared to the invalid subset of the DCA data sets.

**14.** The method of any one of claims 10 to 13, further comprising tuning the CNN model by inputting, into a layer of the CNN model, at least one of i) age-related feature, ii) gender, iii) body mass index, iv) ethnicity, v) medical history, vi) medication usage, vii) lifestyle factor, or viii) family history.

**15.** The method of any one of claims 10 to 14, wherein the CNN model comprises at least two layers, the method further comprising:

freezing one of the at least two layers; and
updating, using additional DCA data sets, at least one layer of the CNN model that is not frozen.

128
120
126
102
100
114
External Instrument
154

FIG. 1A

100
160
Memory
162
121
Programmable Microcontroller
134
Arrhythmia Detector
136
ORI Process
140
Communication Modem
154
External Device
166
164
Telemetry Circuit
170
Physiological Sensor(s)
172
Battery
146
144
Sense
156
150
A/D
127
Elect. Config. Switch
102
113
115

FIG. 1B

200
208
219
218
212
209
216
214

FIG. 2A

212
218
200
288
Memory
262
220
Programmable Microcontroller
224
222
Pulse Gen.
226
214
External Device
264
Telemetry Circuit
232
Timing Control
246
244
Sense
252
270
Physiological Sensor(s)
234
Arrhythmia Detector
228
254
Elect. Config. Switch
282
280
Shocking Circuit
256
272
Battery
236
Morphology Detector
258
286
284
A/D DAS
260
274
Impedance Measuring Circuit
240
Communication Modem

FIG. 2B

300
302
IMD
PATIENT
SMARTPHONE
304
306
Remote Monitoring
Data Server
AI based EGM
Classification
308
CLINICIAN
PORTAL

FIG. 3

420
1D Input
402
400
403a
Batch norm
Activation: ReLU
Dropout: 0.5
403b
Batch norm
Activation: ReLU
Dropout: 0.5
403c
Batch norm
Activation: ReLU
Dropout: 0.5
403d
Batch norm
Activation: ReLU
Dropout: 0.5
403e
Batch norm
Activation: ReLU
Dropout: 0.5
Convolutional Block 1
(filters:64, kernel: 33x1)
C1 410
Convolutional Block 2
(filters:64, kernel: 33x1)
C2 412
Convolutional Block 3
(filters:64, kernel: 33x1)
C3 414
Convolutional Block 4
(filters:64, kernel: 33x1)
C4 416
Convolutional Block 5
(filters:64, kernel: 33x1)
C5 418
404
Global Ave. Pooling
406
408
Softmax
Dense Layer

FIG. 4A

Pause
Amplitude (mv)
Time(s)
Pause
Amplitude (mv)
Trigger
Time(s)

FIG. 4B

FIG. 4C

430
432
Fully connected (FC)
Global Average Pooling (GAP)
flatten

FIG. 4D

| Name | Range | Type |
|---|---|---|
| Activation | (ReLU, ELU) | Choice |
| Dropout | (0,0.9) | Continuous |
| Kernel size | (3,5,7,...,55) | Choice |
| Learning rate | (0.0001,0.001,0.01,0.1) | Choice |
| Learning rate Delay | (0.5,1) | Continuous |
| Number of dense nodes or GAP | (8,128,512) | Discrete |
| Number of filters | (16,32,64,128) | Choice |
| Optimizer | (Adam,SGD and RMSprop) | Choice |
| Regularization | (l1,l2,l1l2),(0.01,0.1) | Choice |

FIG. 4E

$$Accuracy = \frac{TP + TN}{TP + TN + FP + FN}$$

$$Specificity = \frac{TN}{TN + FP}$$

$$Sensitivity = \frac{TP}{TP + FN}$$

$$Precision = \frac{TP}{TP + FP}$$

$$Recall = \frac{TP}{TP + FN}$$

$$PPV = \frac{TP}{TP + FP}$$

$$NPV = \frac{TN}{TN + FN}$$

FIG. 4F

Obtain Collection of Reference DCA Data Sets
502
Augment Collection of Reference DCA Data Sets
504
Sufficient Amount of Reference and Augmented DCA Data Sets?
506
No
Yes
Input Reference and Augmented DCA Data Sets into the CNN Model
508
Train CNN Model
510
Save CNN Model
512
514
Generate Personalized CNN Model
516
Train New CNN Model Using Transfer Learning


**FIG. 5**

Obtain CNN Model
602
Obtain Collection of New DCA Data Sets
604
Apply Current DCA Data Set to CNN Model to Obtain Valid/Invalid Indicator
606
Indicator Exceed Threshold?
608
No
Yes
616
610
612
Move to Next DCA Data Set
Add DCA Data Set to Valid Subset
Add DCA Data Set to Invalid Subset
614
Yes
More DCA Data Sets to Review?
No
Present Information for Valid Subset to Clinician
618
Provide Treatment
620

FIG. 6A

Obtain One or More DCA Data Sets
652
Apply DCA Data Set(s) to CNN Model to Obtain Valid/Invalid Indicator
654
656
Indicator Exceed Threshold?
No
Yes
658
Add DCA Data Set to Valid Subset
660
Add DCA Data Set to Invalid Subset
662
Provide Treatment


FIG. 6B

703
IMD
700
714
Cell Phone
716
Local Monitoring Device
710
User Workstation
702
Server
712
Internet
704
Database
708
Local Monitoring Device
706
Programmer
724
720
Surface ECG Unit
703
IMD
722
ECG Lead
703
IMD

FIG. 7

808
Bedside Monitor
810
802
Doctor
804
806

FIG. 8

# EUROPEAN SEARCH REPORT

Application Number
EP 24 18 4149

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Lashgari Elnaz ET AL: "A CONVOLUTIONAL NEURAL NETWORK FOR AUTOMATIC DISCRIMINATION OF PAUSE EPISODES DETECTED BY AN INSERTABLE CARDIAC MONITOR", Cardiovascular Digital Health Journal, 31 August 2022 (2022-08-31), XP093228249, Retrieved from the Internet: URL:https://www.cvdigitalhealthjournal.com/article/S2666-6936(22)00050-0/fulltext [retrieved on 2024-11-26] | 1-13,15 | INV. G16H50/20 G16H15/00 A61B5/00 G16H20/40 G16H40/40 G16H40/67 G16H50/30 G16H50/70 |
| Y | * Left column, Section "A CONVOLUTIONAL NEURAL NETWORK FOR AUTOMATIC DISCRIMINATION OF PAUSE EPISODES DETECTED BY AN INSERTABLE CARDIAC MONITOR" * ----- | 3-7, 12-14 | |
| Y | US 2022/117538 A1 (DAVIS KEVIN J [US] ET AL) 21 April 2022 (2022-04-21) | 3-7, 12-14 | |
| A | * paragraph [0066] * * paragraph [0093] * * paragraph [0106] * * figure 7 * ----- | 1,2, 8-11,15 | |
| | | | **TECHNICAL FIELDS SEARCHED (IPC)** |
| T | Anonymous: "DEEP LEARNING-BASED DISCRIMINATION OF PAUSE EPISODES IN INSERTABLE CARDIAC MONITORS", , 21 September 2023 (2023-09-21), pages 1-8, XP093228259, Retrieved from the Internet: URL:https://openreview.net/pdf/3cc711ed21529a24a640b379196e95aa3ae1629f.pdf [retrieved on 2024-11-26] * the whole document * ----- | 1-15 | G16H A61B |

1

The present search report has been drawn up for all claims

| Place of search | Date of completion of the search | Examiner |
|---|---|---|
| Munich | 26 November 2024 | Bonnet, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 24 18 4149

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2024

| Patent document cited in search report | Publication date | Patent family member(s) | Publication date |
|---|---|---|---|
| US 2022117538 A1 | 21-04-2022 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8135456 B
- US 11559241 B
- US 11633141 B
- US 7294108 B
- US 9949660 B
- US 9216285 B
- US 8391980 B
- US 20220117538 A
- US 20220304612 A
- US 8831747 B
- US 9232485 B
- US 10765860 B
- US 10722704 B
- US 11045643 B
- US 10729346 B
- US 11020036 B
- US 10874322 B
- US 10777880 B
- US 20210020294 A